# EUROPEAN PATENT SPECIFICATION

(11) **EP 3 189 140 B1**
(45) Date of publication and mention of the grant of the patent: **23.10.2019**
(21) Application number: 15774716.3
(22) Date of filing: 04.09.2015
(51) Int. Cl.: C12N 15/11, C12N 15/113, C07K 14/315, C12N 9/22

(54) **PROGRAMMABLE RNA SHREDDING BY THE TYPE III-A CRISPR-CAS SYSTEM OF STREPTOCOCCUS THERMOPHILUS**
PROGRAMMIERBARE RNA-ZERKLEINERUNG DURCH DAS TYP III-A-CRISPR-CAS-SYSTEM VON STREPTOCOCCUS THERMOPHILUS
DÉFIBRAGE PROGRAMMABLE DE L'ARN PAR LE SYSTÈME CAS-CRISPR DE TYPE III-A DE STREPTOCOCCUS THERMOPHILUS

(30) Priority: 05.09.2014 US 201462046384 P
(43) Date of publication of application: 12.07.2017
(73) Proprietor: Vilnius University, 01513 Vilnius (LT)
(72) Inventor: SIKSNYS, Virginijus, LT-08114 Vilnius (LT); KAZLAUSKIENE , Migle, LT-05254 Vilnius (LT); TAMULAITIS, Gintautas, LT-06203 Vilnius (LT)
(74) Representative: Petniunaite, Jurga
(86) International application number: PCT/IB2015/056756
(87) International publication number: WO 2016/035044

(56) References cited:
- WO-A1-2012/164565
- WO-A2-2007/025097
- US-A1- 2010 076 057
- STAALS RAYMOND H J ET AL: "Structure and Activity of the RNA-Targeting Type III-B CRISPR-Cas Complex ofThermus thermophilus", MOLECULAR CELL, vol. 52, no. 1, 10 October 2013 (2013-10-10), pages 135-145, XP028737307, ISSN: 1097-2765, DOI: 10.1016/J.MOLCEL.2013.09.013 cited in the application
- A. HATOUM-ASLAN ET AL: "Genetic Characterization of Antiplasmid Immunity through a Type III-A CRISPR-Cas System", JOURNAL OF BACTERIOLOGY, vol. 196, no. 2, 15 January 2014 (2014-01-15), pages 310-317, XP055233865, US ISSN: 0021-9193, DOI: 10.1128/JB.01130-13 cited in the application
- A. HATOUM-ASLAN ET AL: "A Ruler Protein in a Complex for Antiviral Defense Determines the Length of Small Interfering CRISPR RNAs", JOURNAL OF BIOLOGICAL CHEMISTRY, vol. 288, no. 39, 27 September 2013 (2013-09-27), pages 27888-27897, XP055233858, US ISSN: 0021-9258, DOI: 10.1074/jbc.M113.499244 cited in the application
- GINTAUTAS TAMULAITIS ET AL: "Programmable RNA Shredding by the Type III-A CRISPR-Cas System of Streptococcus thermophilus", MOLECULAR CELL., vol. 56, no. 4, 6 November 2014 (2014-11-06), pages 506-517, XP055233868, US ISSN: 1097-2765, DOI: 10.1016/j.molcel.2014.09.027
- RAYMOND H.J. STAALS ET AL: "RNA Targeting by the Type III-A CRISPR-Cas Csm Complex of Thermus thermophilus", MOLECULAR CELL., vol. 56, no. 4, 6 November 2014 (2014-11-06), pages 518-530, XP055233869, US ISSN: 1097-2765, DOI: 10.1016/j.molcel.2014.10.005

## Description

Immunity against viruses and plasmids provided by CRISPR-Cas systems relies on a ribonucleoprotein effector complex that triggers degradation of invasive nucleic acids (NA). Effector complexes of Type I (Cascade) and II (Cas9-dual RNA) target foreign DNA. Genetic evidence suggests that Type III-A Csm complex targets DNA, whereas biochemical data show that III-B Cmr complex cleaves RNA.

Disclosed is NA specificity and mechanism of CRISPR-interference for the *Streptococcus thermophilus* Csm (III-A) complex (StCsm). When expressed in *Escherichia coli,* two complexes of different stoichiometry co-purified with 40- and 72-nt crRNA species, respectively. Both complexes targeted RNA and generated multiple cuts at 6 nucleotide (nt) intervals. The Csm3 protein, present in multiple copies in both Csm complexes, acts as endoribonuclease. In the heterologous *E. coli* host StCsm restricts MS2 RNA phage in Csm3 nuclease-dependent manner.

As subsequently disclosed in detail, the inventors determined that *Streptococcus thermophilus* Type III-A Csm (StCsm) complex targets RNA. The inventors also determined that multiple cuts are introduced in the target RNA at 6 nt intervals. Target RNA that is complimentary to crRNA is cleaved at multiple sites at regular 6 nt intervals, also termed shredding. RNA cleavage is protospacer-adjacent motif (PAM) independent. A Csm3 subunit is responsible for endoribonuclease activity of the complex. Because multiple copies of Csm3 subunits are present in the Csm complex, cleavage occurs at multiple sites. By systematic deletion of the genes encoding individual subunits, the minimal Csm complex composition required for target RNA cleavage was established.

The StCsm complex offers a novel programmable tool for RNA-degradation or modification, e.g., in methods similar to RNA Interference (RNAi) methods, and using RNAi methods known in the art. However, different from RNA interference based methods that rely on the RNAi binding to the target RNA resulting in the gene product knock-down, RNA-targeting by the Csm complex allows knock-out of the gene product because the target RNA is cleaved at multiple sites. If an RNA-cleavage deficient Csm complex (Csm3D33A) is used, knock-downs instead of knockouts can be achieved, which provides additional flexibility.

Clustered Regularly Interspaced Short Palindromic Repeats (CRISPR) together with Cas (CRISPR-associated) proteins provide RNA-mediated adaptive immunity against viruses and plasmids in bacteria and archaea (Terns and Terns, 2014). Immunity is acquired through the integration of invader-derived nucleic acid (NA) sequences as 'spacers' into the CRISPR locus of the host. CRISPR arrays are further transcribed and processed into small interfering CRISPR RNAs (crRNAs) that together with Cas proteins assemble into a ribonucleoprotein (RNP) complex which, using crRNA as a guide, locates and degrades the target NA. CRISPR-Cas systems have been categorized into three major Types (I-III) that differ by the structural organization of RNPs and NA specificity (Makarova et al., 2011b).

Type I and II systems provide immunity against invading DNA. In Type I-E systems, crRNAs are incorporated into a multisubunit RNP complex called Cascade (CRISPR-associated complex for antiviral defense) that binds to the matching invasive DNA and triggers degradation by the Cas3 nuclease/helicase (Brouns et al., 2008; Sinkunas et al., 2013; Westra et al., 2012). In Type II systems, CRISPR-mediated immunity solely relies on the Cas9 protein. It binds a dual RNA into the RNP effector complex, which then specifically cuts the matching target DNA, introducing a double strand break (Gasiunas et al., 2012; Jinek et al., 2012). In Type I and II CRISPR-Cas systems, the target site binding and cleavage requires a short nucleotide sequence (protospacer-adjacent motif, or PAM) in the vicinity of the target (Mojica et al., 2009). Target DNA strand separation, necessary for the crRNA binding, is initiated at PAM and propagates in a directional manner through the protospacer sequence to yield the R-loop intermediate, one strand of which is engaged into the heteroduplex with crRNA, while the other strand is displaced into solution (Sternberg et al., 2014; Szczelkun et al., 2014). Thus, despite differences in their architecture, Type I and II RNP complexes share three major features: i) they act on the invasive double-stranded DNA (dsDNA), e.g., viral DNA or plasmids, ii) they require the presence of a PAM sequence in the vicinity of the target site, and iii) they generate an R-loop as a reaction intermediate.

Type III CRISPR-Cas systems are believed to target either DNA (Type III-A) or RNA (Type III-B) (Makarova et al., 2011b). In the III-B systems Cas RAMP proteins (Cmr) and crRNA assemble into a multisubunit RNP complex. Using crRNA as a guide, this complex *in vitro* binds single-stranded RNA (ssRNA) in a PAM-independent manner and triggers the degradation of target RNA (Hale et al., 2009; Staals et al., 2013; Zhang et al., 2012). The Cmr effector complex is comprised of six Cmr proteins (Cmr1, Cas10, Cmr3-6) that are important for target RNA cleavage; however roles of the individual Cmr proteins and the ribonuclease (RNase) component have yet to be identified. Cmr1, Cmr3, Cmr4 and Cmr6 are predicted RNA-binding proteins that share a ferredoxin-like fold and RNA-recognition motif (RRM) identified in RNA-binding proteins (Terns and Terns, 2014).

The *cas* genes encoding the RNA-targeting Type III-B (Cmr) and DNA-targeting Type III-A (Csm) effector complexes share a partial synergy (Makarova et al., 2011a). In *Staphylococcus epidermidis* the Csm complex (SeCsm) is comprised of Cas10, Csm2, Csm3, Csm4, and Csm5 proteins, however the function of individual Csm proteins is unknown. The evidence that Type III-A systems target DNA remains indirect and relies on the experimental observation that Type III-A RNP complex from *Staphylococcus epidermidis* (SeCsm) limits plasmid conjugation and transformation *in vivo,* but the DNA degradation has not been demonstrated directly (Marraffini and Sontheimer, 2008, 2010). The Csm complex from the archaeon *Sulfolobus solfataricus* (SsCsm) binds dsDNA, however, it shows no crRNA-dependent nuclease activity *in vitro* (Rouillon et al., 2013). Thus, while the RNA cleavage activity of the Cmr complex has been characterized *in vitro,* the DNA degradation activity of the Type III-A Csm complex has yet to be demonstrated. The Csm complex so far remains the only CRISPR-Cas effector complex, for which the function is not yet reconstituted *in vitro.* The inventors established the composition and mechanism of the Csm complex for Type III-A system *Streptococcus thermophilus* (St) and demonstrated its RNA cleavage activity *in vitro* in the cell.

### BRIEF DESCRIPTION OF THE FIGURES

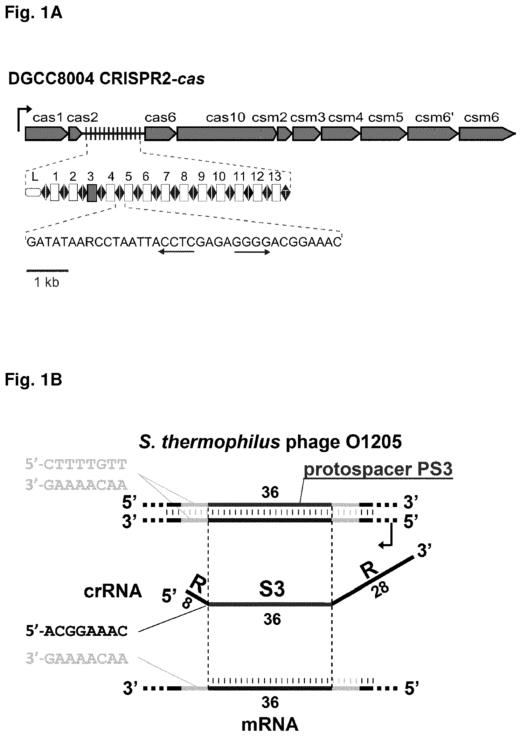

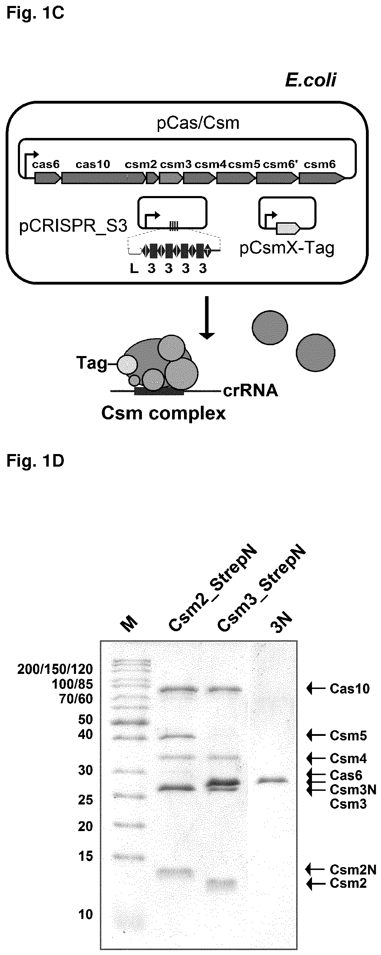

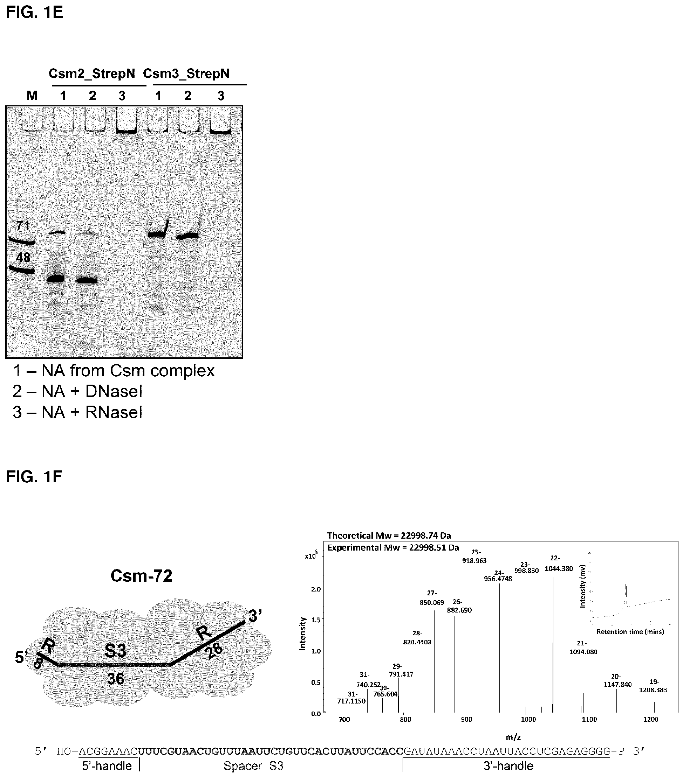

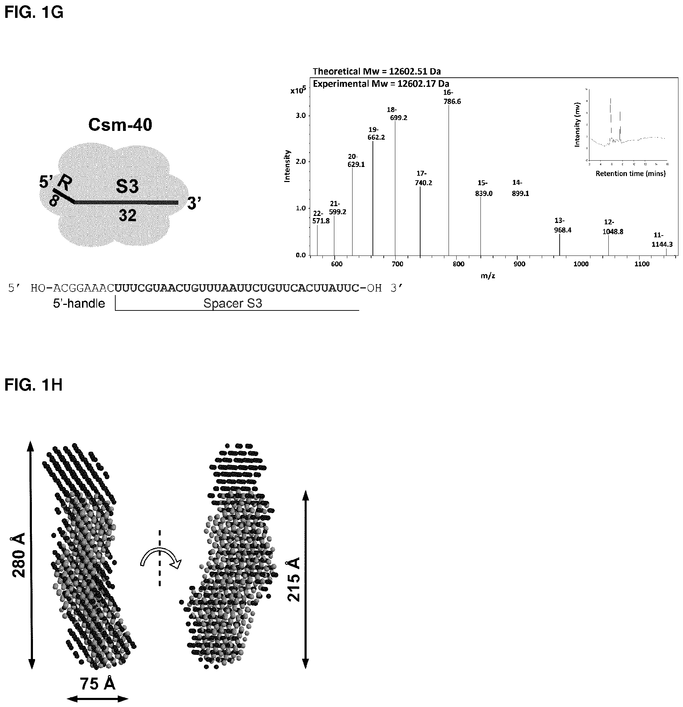

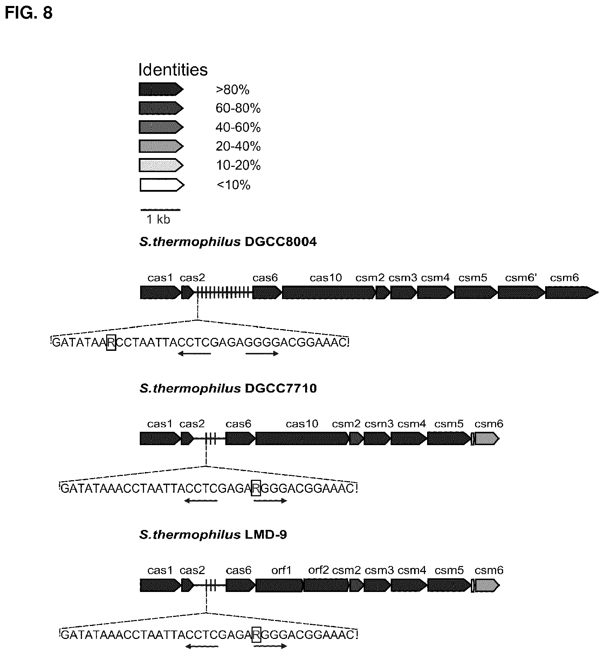

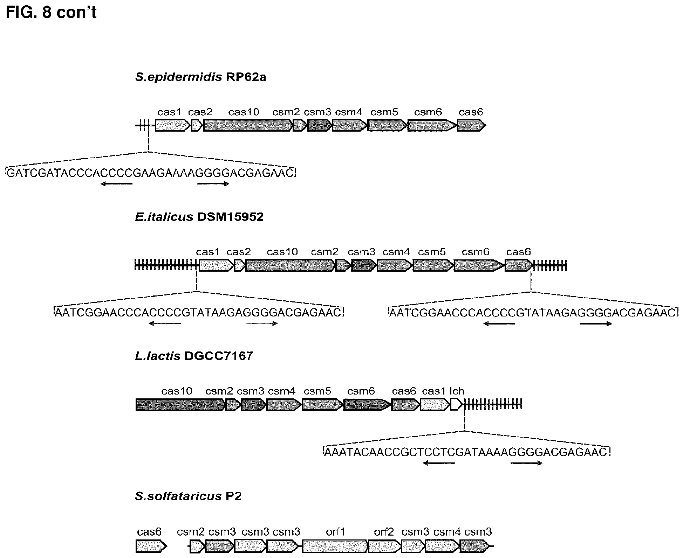

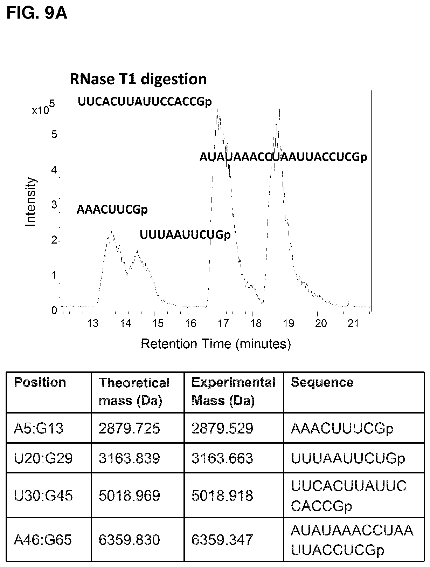

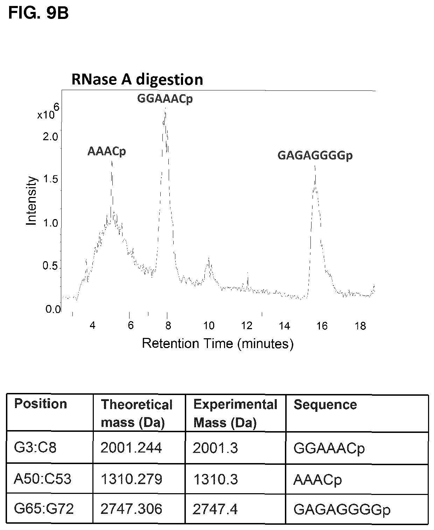

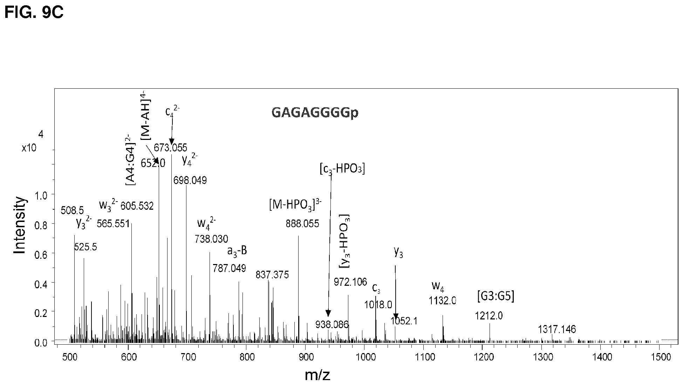

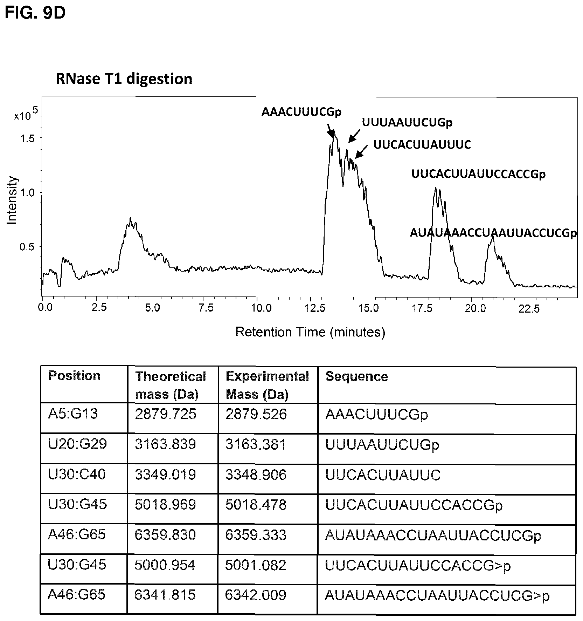

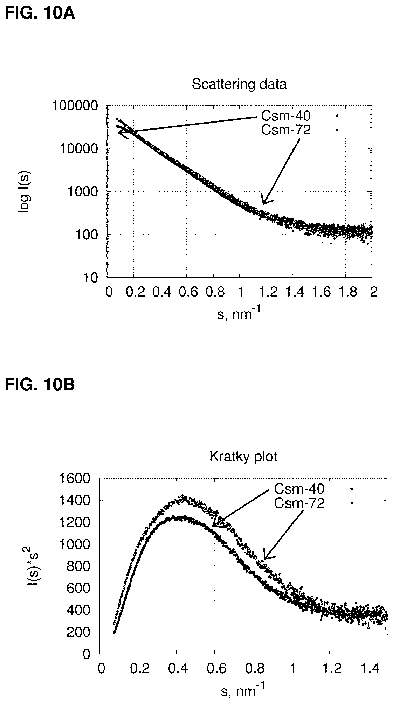

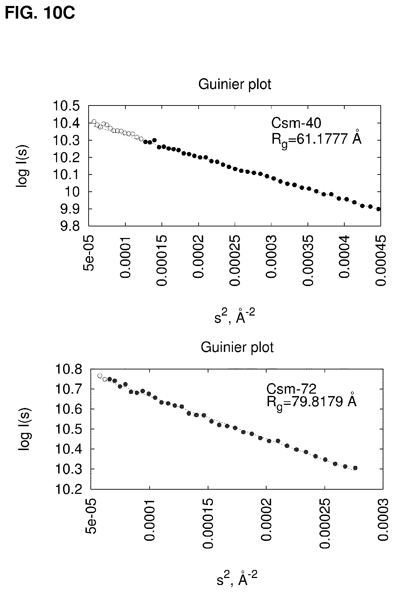

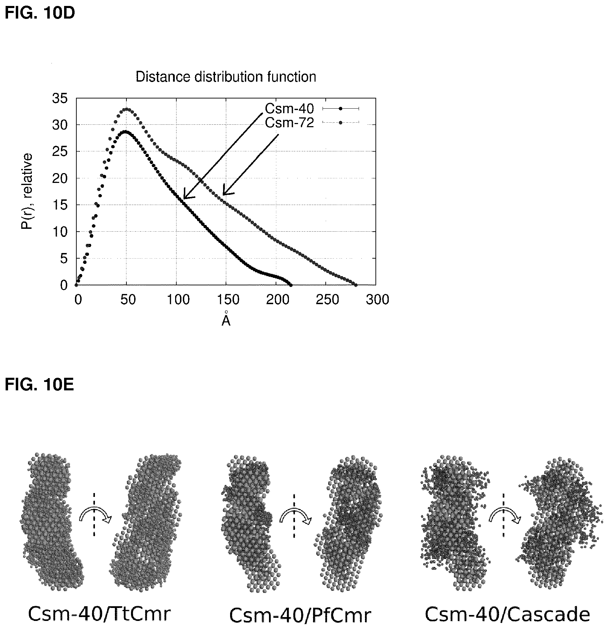

FIG. 1 shows cloning, isolation and characterization of the Type III-A Csm complex of S. *thermophilus* DGCC8004. (A) Schematic organization of the Type III-A CRISPR2-cas locus (see also FIG. 8). Repeats and spacers are indicated by diamonds and rectangles, respectively, T is for the terminal repeat, L is for the leader sequence, and the arrow indicates the promoter. (B) Protospacer PS3 and the 5'-flanking sequence in the *S. thermophilus* phage 01205 genome. (C) Strategy for expression and isolation of the StCsm complex. Four copies of the spacer S3 have been engineered into the pCRISPR_S3 plasmid to increase the yield of the Csm-crRNA complex. (D) Coomassie blue-stained SDS-PAAG of Strep-tagged Csm2 and Csm3 pull-downs. 3N - Csm3_StrepN protein, M - protein mass marker. (E) Denaturing PAGE analysis of NA co-purifying with the Csm2_StrepN and Csm3_StrepN complexes. M - synthetic DNA marker. (F) and (G) Characterization of crRNA in the isolated StCsm complexes. Cartoon models illustrate crRNA which co-purify with StCsm-72 and StCsm-40 complexes. Composition of the crRNA was determined using LC ESI MS analysis (see also FIG. 9). IP RP HPLC analysis and ESI MS spectra of IP RP HPLC purified crRNA from StCsm-40 and StCsm-72 are presented. (H) Superimposed averaged dummy atom models obtained from SAXS data of StCsm-40 (light beads) and StCsm-72 (dark beads) (see also FIG. 10).

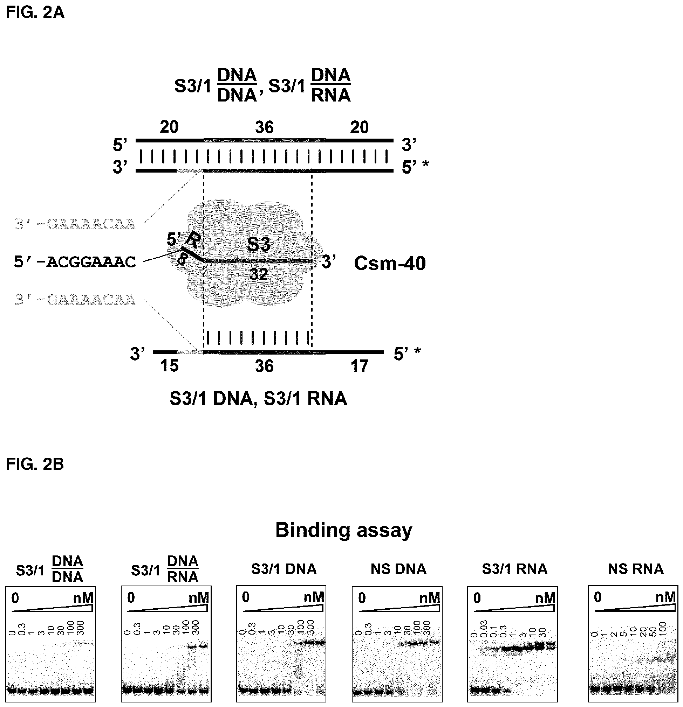

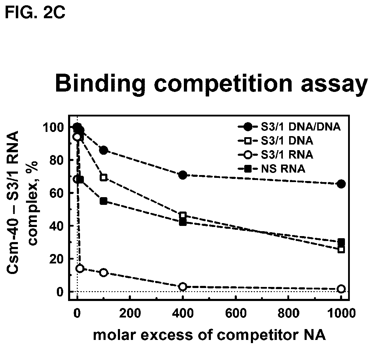

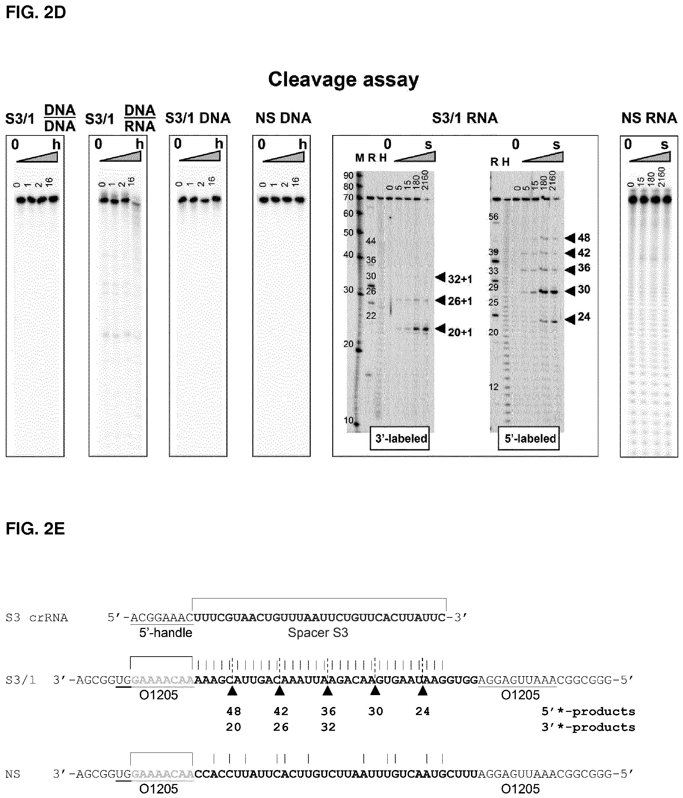

FIG. 2 shows nucleic acid binding and cleavage by the Type III-A Csm complex of *S. thermophilus.* (A) Schematic representation of DNA and RNA substrates used for *in vitro* binding and cleavage assays. NAs were 5'- or 3'-end labeled with ³²P (indicated as *). (B) EMSA analysis of DNA or RNA binding by StCsm-40. NS stands for a non-specific RNA. (C) Binding competition assay. 0.5 nM of ³²P-labelled S3/1 RNA was mixed with increasing amounts of unlabelled competitor NAs and 0.3 nM StCsm-40, and analyzed by EMSA. (D) StCsm-40 cleavage assay. Gel-purified DNA or RNA were used as substrates in the NA cleavage assay. Triangles with corresponding numbers indicate cleavage product length. M - RNA Decade marker, R - RNase A digest marker, H - alkaline hydrolysis marker. (E) RNA cleavage products mapped on the S3/1 RNA substrate sequence. Triangles and dashed lines indicate cleavage positions. Short vertical lines above the sequence indicate nucleotides complementary to crRNA. crRNA (StCsm-40) sequence is depicted above the matching substrate fragments.

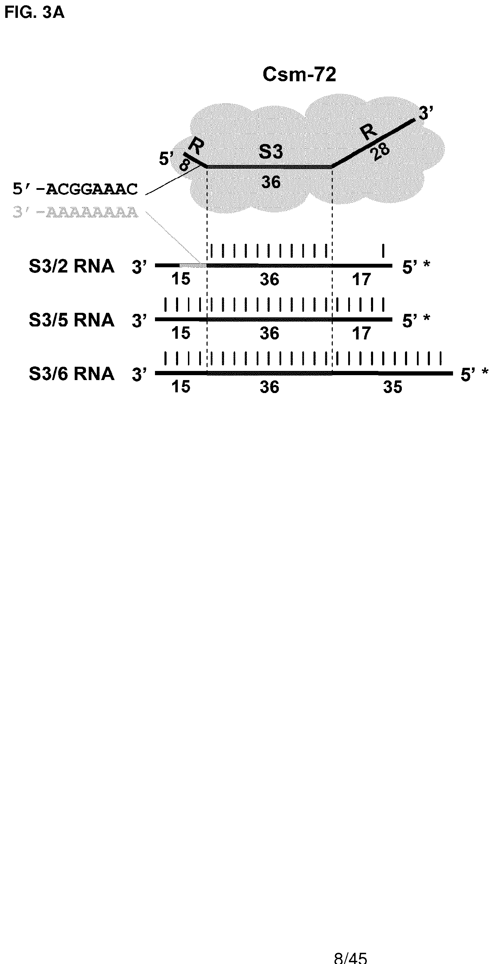

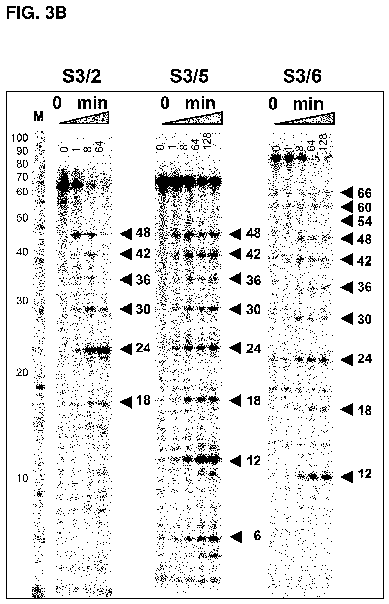

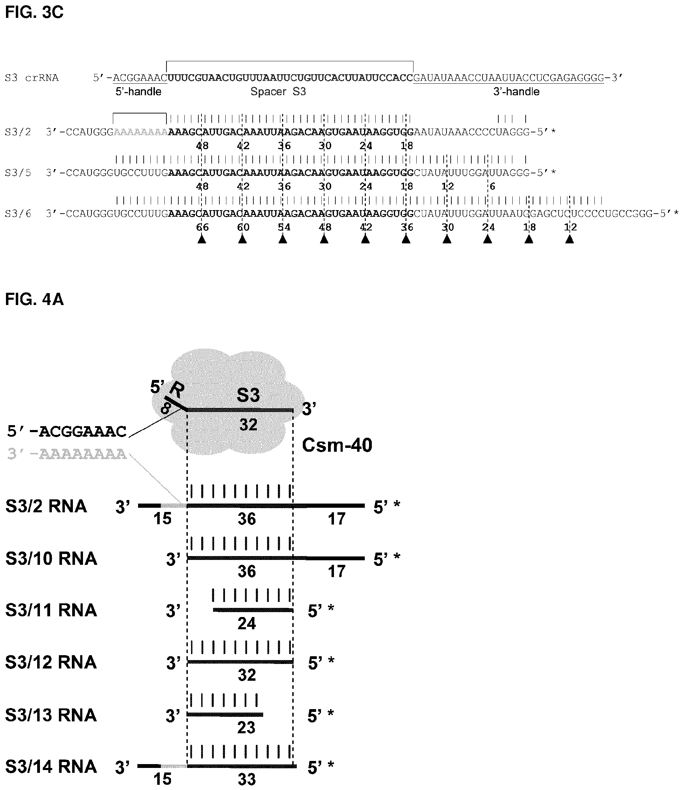

FIG. 3 shows the effect of the sequence complementarity outside the spacer region on the StCsm-72 cleavage pattern. (A) Schematic representation of the StCsm-72 complex and RNA substrates used in the cleavage assay. RNA substrates were 5'-end labeled with ³²P (indicated as *) and gel-purified. (B) StCsm-72 cleavage assay. M - RNA Decade marker. (C) RNA cleavage products mapped on the S3/2, S3/5 and S3/6 RNA substrates sequences.

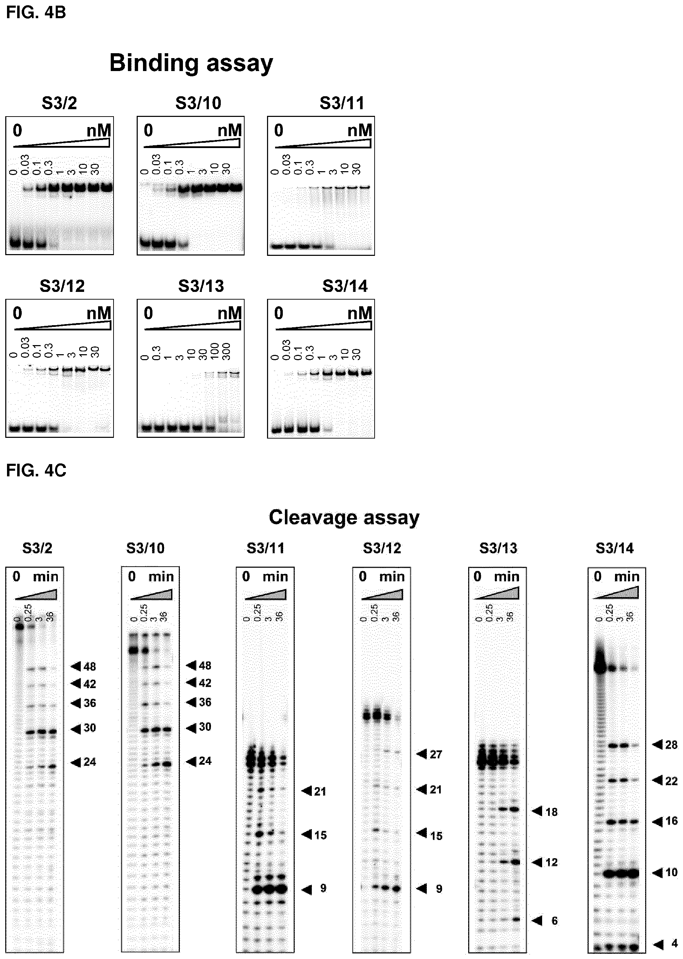

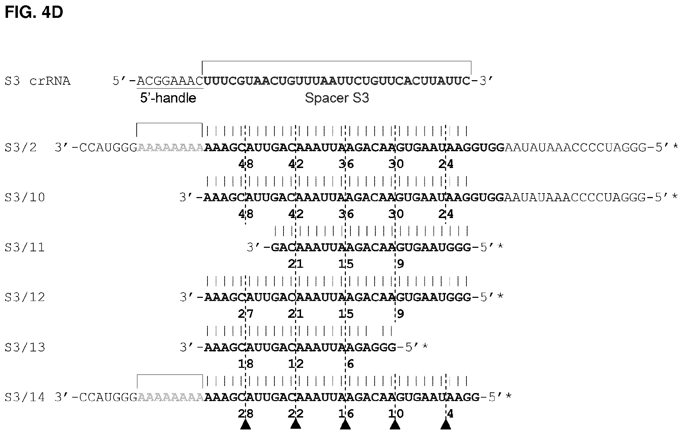

FIG. 4 shows the effect of protospacer truncations on the StCsm-40 cleavage pattern. (A) Schematic representation of the StCsm-40 complex and RNA substrates used in the cleavage assay. RNA substrates were 5'-end labeled with ³²P (indicated as *) and gel-purified. (B) StCsm-40 cleavage assay. M - RNA Decade marker. (C) RNA cleavage products mapped on the RNA substrates sequences.

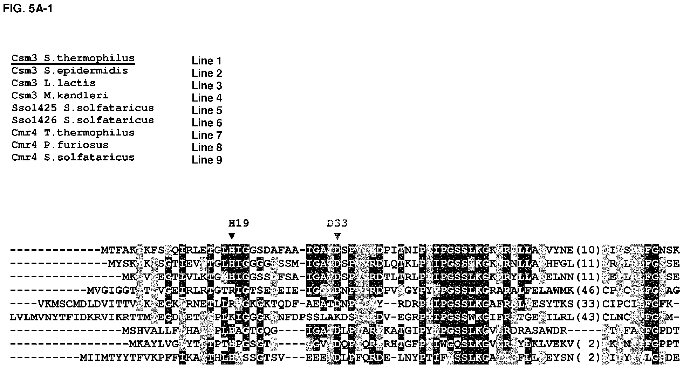

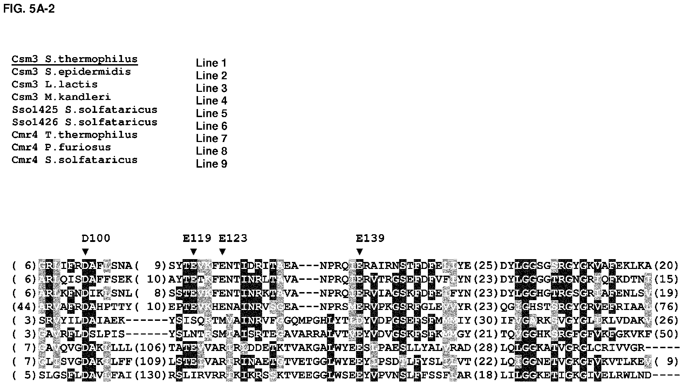

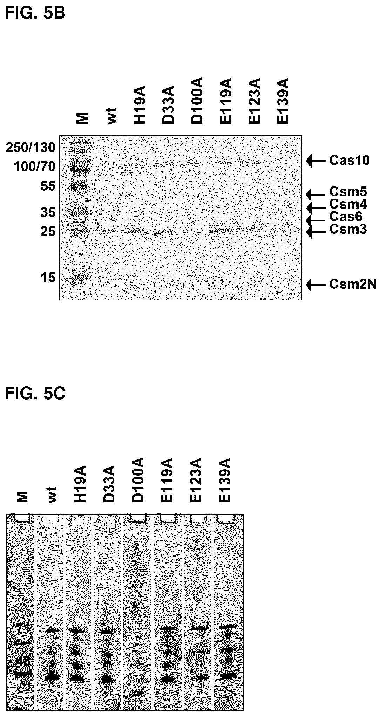

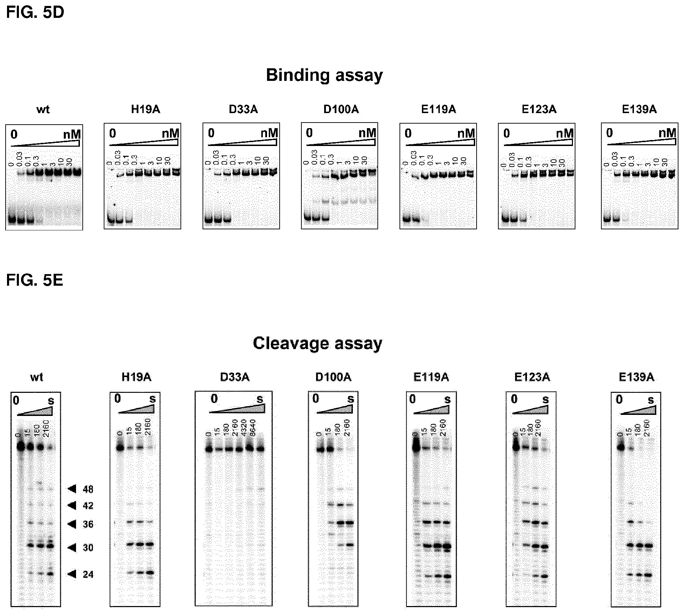

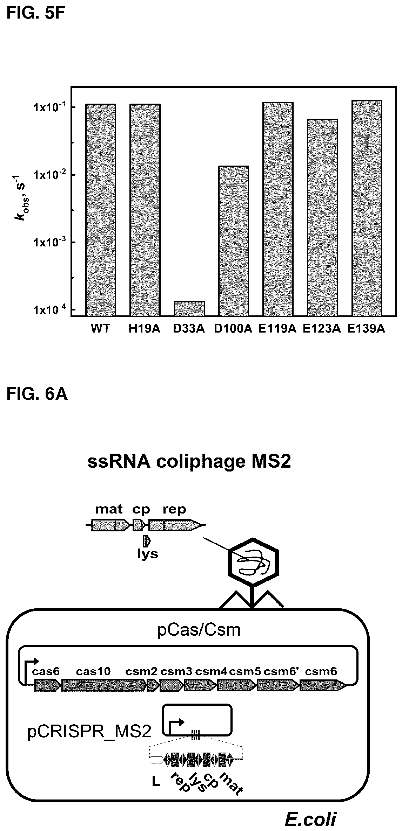

FIG. 5 shows computational and mutational analysis of Csm3. (A) Alignment of Csm3 and Cmr4 sequence representatives from experimentally characterized Type III effector complexes. Identical and similar residues in more than half of sequences are shaded in dark and light correspondingly. StCsm3 positions subjected to site-directed mutations are indicated by triangles above the sequence. (B) Coomassie blue-stained SDS-PAAG of StCsm complexes containing Csm3 mutants. M - protein marker. (C) Denaturing PAGE analysis of NA co-purifying with mutant StCsm complexes. M - synthetic DNA marker. (D) EMSA analysis of S3/2 RNA binding by mutant StCsm complexes. (E) S3/2 RNA cleavage by the mutant StCsm complexes. (F) The cleavage rate constant k_{obs} values for Csm3 mutant variants of StCsm-40.

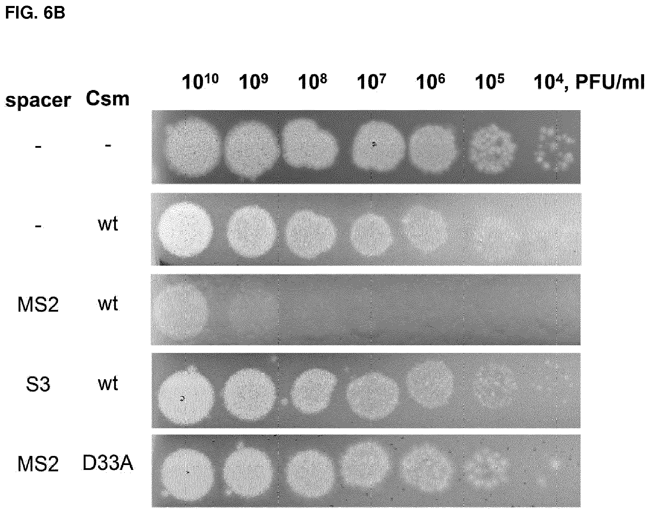

FIG. 6 shows restriction of ssRNA phage MS2 in *E. coli* cells expressing StCsm complex. (A) Schematic representation of the assay. The arrow indicates the promoter. (B) Phage plaque analysis. Serial 10-fold dilutions of MS2 were transferred onto lawns of *E. coli* NovaBlue (DE3, F⁺) strain expressing StCsm-crRNA complex targeting the MS2 genome, or control cells.

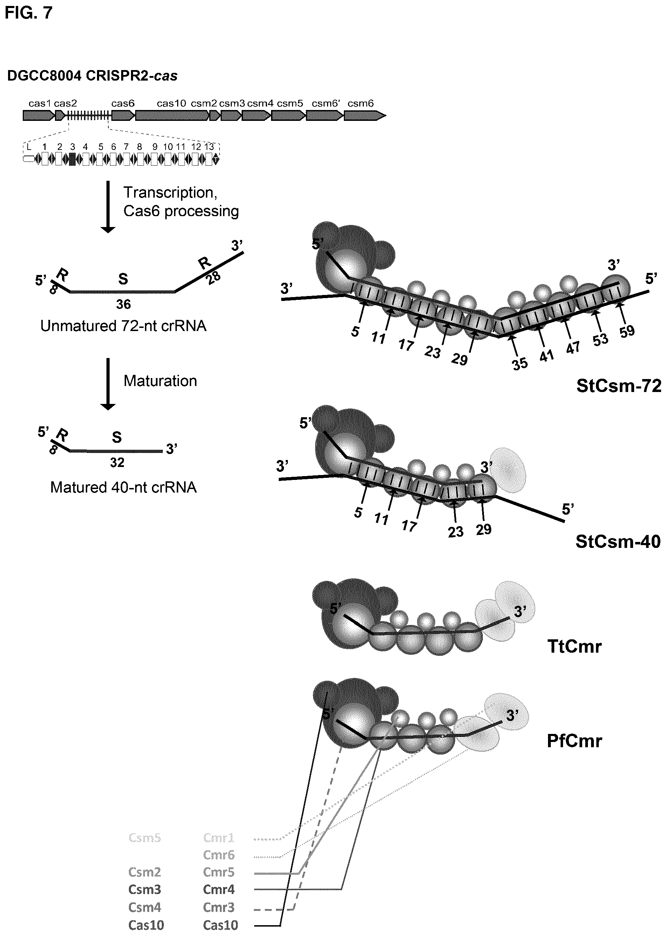

FIG. 7 shows structural and cleavage models of StCsm complexes. The CRISPR2 transcript is first processed into 72-nt crRNA intermediates which undergo further maturation into 40-nt crRNA. Both crRNAs are incorporated into StCsm complexes that target RNA but differ by the number of Csm3 and Csm2 subunits. The number of RNA cleavage products correlate with the number of Csm3 nuclease subunits. Schematic models of StCsm complexes were generated based on similarity to TtCmr and PfCmr. Csm analogs of Cmr proteins according to Makarova et al., 2011a are shaded the same, and as indicated.
FIG. 8 shows schematic organization of the Type III-A CRISPR-Cas systems of *Streptococcus thermophilus* DGCC8004, DGCC7710, LMD-9, *Staphylococcus epidermidis* RP62a, *Enterococcus italicus* DSM15952, *Lactococcus lactis* DGCC7167 and *Sulfolobus solfataricus* P2. Schematic organization of the Type 1II-A CRISPR-Cas systems of *Streptococcus thermophilus* DGCC8004 (GenBank KM222358), DGCC7710 (GenBank AWVZ01000003), LMD-9 (GenBank NC008532), *Staphylococcus epidermidis* RP62a (GenBank NC002976), *Enterococcus italicus* DSM15952 (GenBank AEPV01000074), *Lactococcus lactis* DGCC7167 (GenBank JX524189) and *Sulfolobus solfataricus* P2 (GenBank AE006641)*. Arrows are shaded according to the percentage of identical residues (Vector NTI AlignX tool) in Csm/Cas proteins in respect to the S. *thermophilus* DGCC8004. Conserved repeat sequences are shown in the inserts. Partially palindromic repeat sequences are indicated by arrows. In *L. lactis* DGCC7167 CRISPR2 system *Ich* gene which shows a partial homology to the relE/parE toxin gene is present instead of *cas2* (Millen et al., 2012). In CRISPR-Cas loci of *S. thermophilus* LMD-9 and S. *solfataricus* P2 *cas10* is split in two open reading frames ORF1 and ORF2. The Type III-A system of DGCC8004 contains 10 *cas* genes flanking the CRISPR2 array and includes *casI, cas2, cas6, cas10, csm2, csm3, csm4, csm5, csm6* and *csm6'* genes. The DGCC8004 CRISPR2 locus share similar gene arrangement to that of DGCC7710 (GenBank AWVZ00000000, (Horvath and Barrangou, 2010)) and LMD-9 (GenBank NC_008532, (Makarova et al., 2006)). The major difference is an additional *csm6'*gene in DGCC8004. The Csm6' protein in DGCC8004 is comprised of 386 aa and shows -34% amino acid identity to the 428 aa Csm6 protein, suggesting a possible ancient gene duplication event followed by sequence divergence. In contrast, DGCC7710 contains only a short 117-nt ORF in front of *csm6.* The Cas/Csm proteins associated to CRISPR2 in DGCC8004 are homologous to the corresponding proteins in DGCC7710 and LMD-9 (more than 90% aa identity, except for the Csm2 protein, which shares ∼70% identity). Other experimentally characterized Type III-A systems including *S. epidermidis* RP62a (GenBank NC002976, (Marraffini and Sontheimer, 2008)), *Enterococcus italicus* DSM15952 (GenBank AEPV01000074, (Millen et al., 2012)) and *Lactococcus lactis* DGCC7167 (GenBank JX524189, (Millen et al., 2012)) share with DGCC8004 a conserved arrangement of the *cas10-csm2-csm3-csm4-csm5-csm6* gene cluster, while the position of *cas6* and *cas1*/*cas2* genes differ in some strains. The Type III-A signature protein Cas10 of DGCC8004 shows ∼34-40% identity (-50-55% similarity) to Cas10 of S. *epidermidis, E. italicus* and *L. lactis.* In LMD-9, the *cas10* gene is split into two ORFs which match to the N- and C-terminal fragments of Cas10 in DGCC8004 (> 92% identical aa). Type III-A CRISPR-Cas locus in *S. solfataricus* P2 (GenBank AE006641) has different gene organization and shows low protein sequence similarity to Cas/Csm orthologues in DGCC8004. Noteworthy, the Csm3 protein is most conserved among the Cas/Csm proteins across different strains and 5 copies of the Csm3 paralogues are present in *S. solfataricus.* Repeat sequences in *S. epidermidis, E. italicus* and *L. lactis* are of the same length (36 nt), however the nucleotide conservation is limited to the palindromic parts and 3'-terminal end of the repeats. The 8-nt 3'-terminal sequence of the repeat, which may contribute to the crRNA 5'-handle, shows an ACGRRAAC consensus between *S. thermophilus, S. epidermidis, E. italicus* and *L. lactis* but differs from that of *S. solfataricus* (AUUGAAG (Rouillon et al., 2013)).
FIG. 9 shows ESI MS/MS oligoribonucleotide mapping of crRNA isolated from StCsm-72 and StCsm-40. ESI MS/MS oligoribonucleotide mapping of crRNA isolated from StCsm-72 and StCsm-40. (A-C) ESI MS/MS oligoribonucleotide mapping of crRNA isolated from StCsm-72. (A) Base peak chromatogram of RNase T1 digest. RNase Ti cleaves single-stranded RNA 3' of G residues. Predominant oligoribonucleotide peaks of the crRNA are highlighted. Masses of each oligoribonucleotide are presented in the table. The theoretical and experimental masses are shown for the oligoribonucleotides identified. (B) Base peak chromatogram of RNase A digest. RNase A cleaves single-stranded RNA 3' of C or U residues. (C) ESI MS/MS analysis of the oligoribonucleotide GAGAGGGGp. Tandem MS was used to verify the oligoribonucleotide. The predominant fragment ions are highlighted. (D) ESI MS/MS oligoribonucleotide mapping of crRNA isolated from StCsm-40. Base peak chromatogram of RNase T1 digest. The oligoribonucleotide UUCACUUAUUC was unique to the 40-nt crRNA. p = 3'-phosphate, >p - ¬,2'3' -cyclic phosphate
FIG. 10 shows SAXS data for StCsm complexes. SAXS data for StCsm-40 (black dots) and StCsm-72 (gray dots) are shown. (A) Scattering profiles shown as a logarithmic plot of scattering intensity I(s) vs momentun transfer s = 4π sin(θ)/λ, where 2θ is the scattering angle and λ is X-ray wavelength. (B) Kratky plot of SAXS data, I(s)*s² *vs s.* (C) Guinier plots of SAXS data, In I(s) *vs s*² and its linear fit. The truncated first points are shown as open circles. (D) Distance distribution functions of StCsm-40 and StCsm-72 complexes calculated using GNOM (Svergun, 1992). (E) The electron density of the TtCmr (dark beads), PfCmr (dark beads), and *E. coli* Cascade complexes (dark beads) aligned with the StCsm-40 (light beads) model.

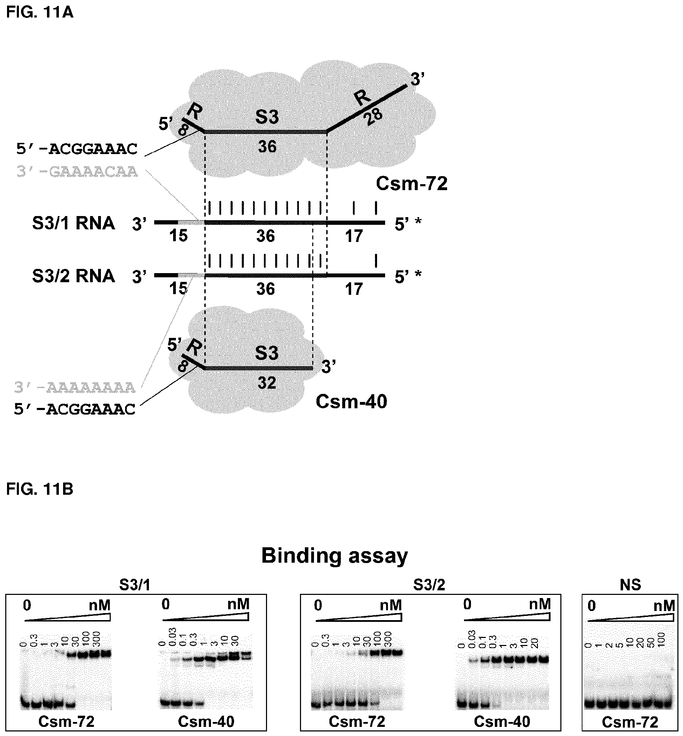

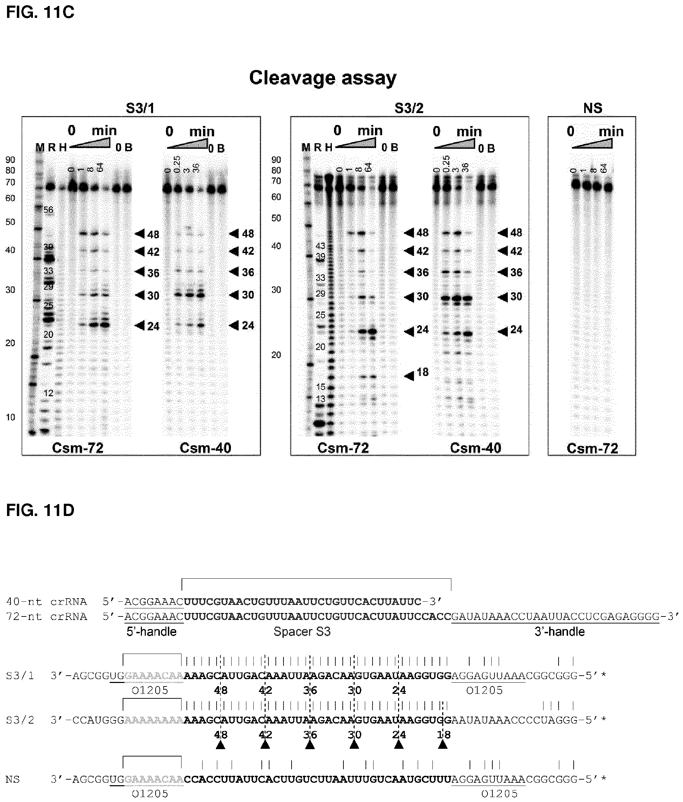

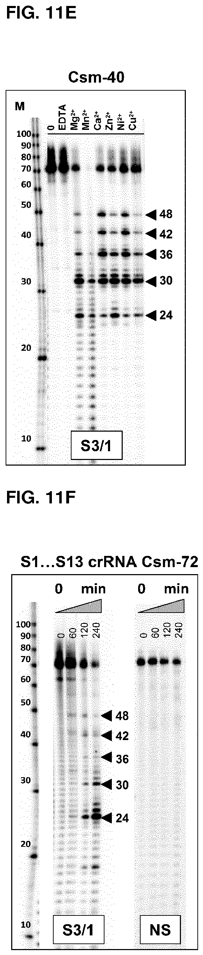

FIG. 11 shows target RNA binding and cleavage by StCsm-72 and StCsm-40 complexes. (A) Schematic representation of S3/1 and S3/2 RNA substrates used in binding and cleavage assays. Nucleic acids were 5'-end labeled with ³²P (indicated as *). (B) Electrophoretic mobility shift binding assay. The binding reactions contained ³²P-labeled RNA (0.5 nM) and the StCsm-72 or StCsm-40 at concentrations indicated by each lane. Samples were analyzed by PAGE under non-denaturing conditions. NS shows the non-specific RNA control.(C) Cleavage assay. Cleavage reactions were performed at 37°C for Csm-72 and 25°C for Csm-40 for indicated time intervals in the Reaction buffer supplemented with 10 mM Mg-acetate, 20 nM RNA substrate and 125 nM StCsm-72 or 62.5 nM StCsm-40. Samples were analyzed by denaturing PAGE, followed by phosphorimaging. In control experiments RNA substrate was incubated for 64 min at 37°C or 36 min at 25°C in the Reaction buffer alone ("lane 0") or the storage buffer was added instead of the Csm complex ("lane B"). Triangles denote the reaction products (the sizes of cleavage fragments are given near triangles). M - RNA Decade marker, R - oligoribonucleotide fragments generated from RNase A digestion of RNA, H - alkaline hydrolysis of RNA. (D) RNA cleavage products mapped on the RNA substrates sequence. Triangles and dashed lines indicate cleavage positions. Short vertical lines above the sequence indicate nucleotides complementary to crRNA. 40-nt and 72-nt crRNAs containing spacer S3 sequences are depicted above the matching substrate fragments. NS stands for non-specific RNA. (E) Metal ion (Me²⁺) dependency of the RNA cleavage by the StCsm complex. S3/1 RNA substrate was pre-incubated with StCsm-40 and reaction products were analyzed in denaturating polyacrylamide gels. Cleavage reactions were conducted at 25°C for 3 min in Reaction buffer containing 20 nM ³²P-5'-labelled gel purified S3/1 RNA substrate, 62.5 nM Csm-40 and 1 mM EDTA, 10 mM Mg-acetate, 10 mM MnC1₂, 1 mM Ca-acetate, 0.1 mM ZnSO₄, 0.1 mM NiC1₂, or 1 mM CuSO₄. Triangles and numbers denote the reaction products and their sizes, respectively. M - RNA Decade marker. (F) S3/1 RNA cleavage pattern of the heterogeneous Csm-complex. To express and to purify heterogeneous Csm complex the wt CRISPR2 region containing 13 spacers of S. *thermophilus* DGCC8004 was cloned into the pACYC-Duet-1 vector. The heterogeneous StCsm-72 complex was expressed and purified following the same procedure described for the homogenous Csm complex targeting the S3 protospacer. Specific S3/1 RNA substrate and non-specific NS RNA were pre-incubated with heterogeneous StCsm-72. Cleavage reactions were performed at 37°C for indicated time intervals in the Reaction buffer supplemented with 10 mM Mg-acetate, 20 nM RNA substrate and 350 nM heterogeneous StCsm-72. Samples were analyzed by denaturing PAGE, followed by phosphorimaging. Triangles and numbers denote the reaction products and their sizes, respectively. M - RNA Decade marker.

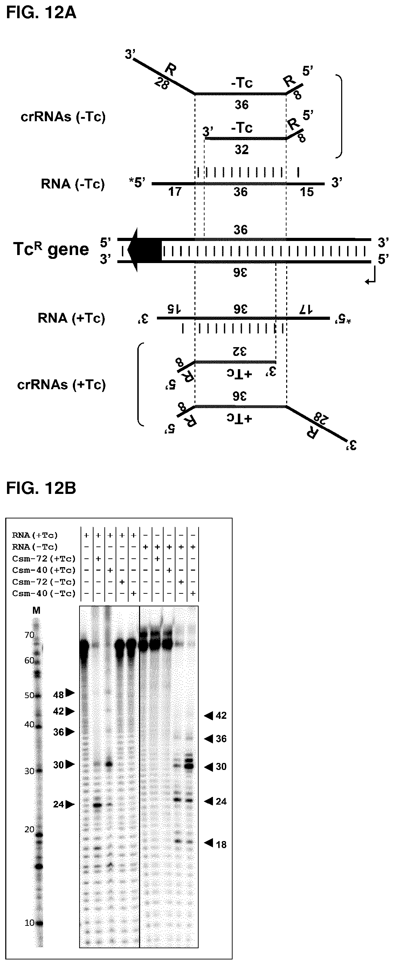

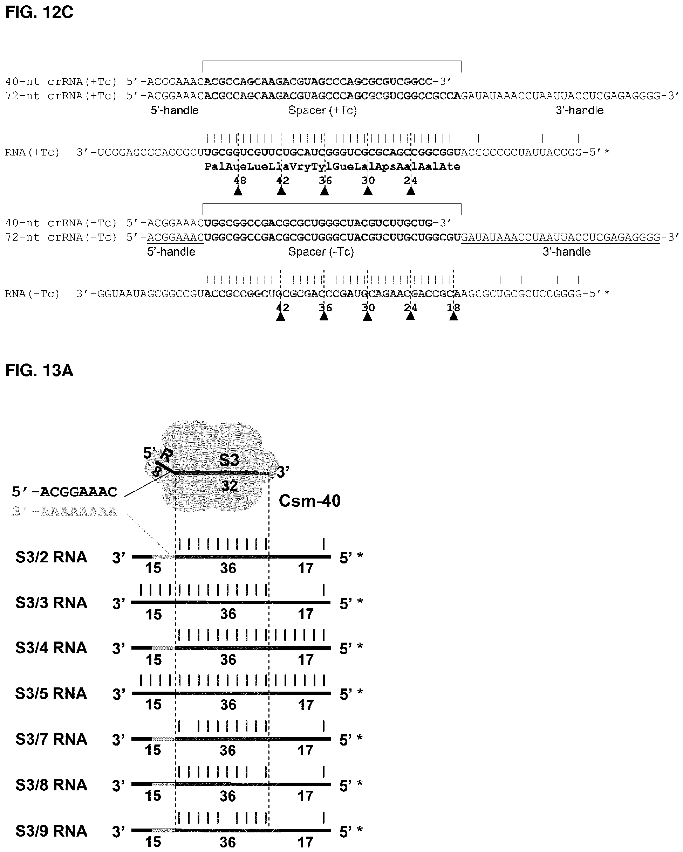

FIG. 12 shows reprogramming of the StCsm complex to cleave a desired RNA. (A) Schematic representation (+Tc) and (-Tc) RNA substrates used in the cleavage assay. Arrows indicate Tc^{R} gene promoter and direction of transcription. RNA substrates were 5'-end labeled with ³²P (indicated as *) and gel purified. The Tc (tetracycline resistance protein) gene transcript or RNA corresponding to the noncoding strand of Tc in the pBR322 plasmid (nt 851-886) were used as RNA targets. To reprogram the StCsm complex a synthetic CRISPR locus containing five 36-nt length repeats interspaced by four identical 36-nt spacers complementary to the sense or antisense DNA strands of the Tc gene were engineered into the pACYC-Duet-1 plasmids which were independently co-expressed in *E. coli* together with plasmids pCsm/Cas and pCsmX-Tag. StCsm-40 and StCsm-72 complexes reprogrammed for the sence (+Tc) RNA or anti-sense (-Tc) RNA fragments were isolated similarly to StCsm bearing spacer S3. (B) Cleavage reactions were performed at 37°C for 120 min in the Reaction buffer supplemented with 10 mM Mg-acetate, 20 nM gel purified RNA substrate and 40-120 nM of Csm complex. Samples were analyzed by denaturing PAGE, followed by phosphorimaging. Triangles with corresponding numbers indicate cleavage product length. M - RNA Decade marker. (C) RNA cleavage products mapped on the (+Tc) and (-Tc) RNA substrates sequences. The sequences of reprogrammed 40-nt and 72-nt length crRNAs are depicted above the substrates. Short vertical lines above the sequence indicate nucleotides complementary to crRNA. Triangles and dashed lines indicate cleavage positions. Translated fragment which corresponds to the tetracycline resistance protein gene RNA transcript is indicated under (+Tc) RNA substrate.

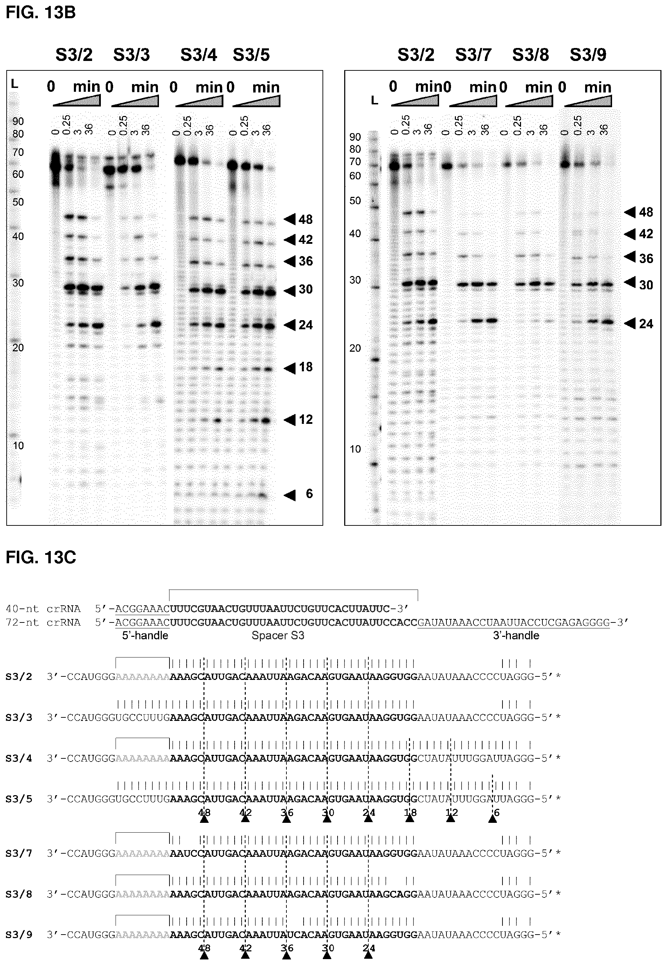

FIG. 13 shows the effect of crRNA:target RNA complementarity on the StCsm-40 cleavage pattern. (A) Schematic representation of the StCsm-40 complex and RNA substrates used in the cleavage assay. RNA substrates were 5' -end labeled with ³²P (indicated as *) and gel purified. (B) Cleavage reactions were performed at 25°C for indicated time intervals in the Reaction buffer supplemented with 10 mM Mg-acetate, 20 nM RNA substrate and 62.5 nM StCsm-40. Samples were analyzed by denaturing PAGE, followed by phosphorimaging. Triangles with corresponding numbers indicate cleavage product length. M - RNA Decade marker. (C) RNA cleavage products mapped on RNA substrates sequences. Short vertical lines above the sequence indicate nucleotides complementary to crRNA. Triangles and dashed lines indicate cleavage positions. The sequences of both 40-nt and 72-nt length crRNAs containing spacer S3 present in StCsm-40 preparation are depicted above the substrates.

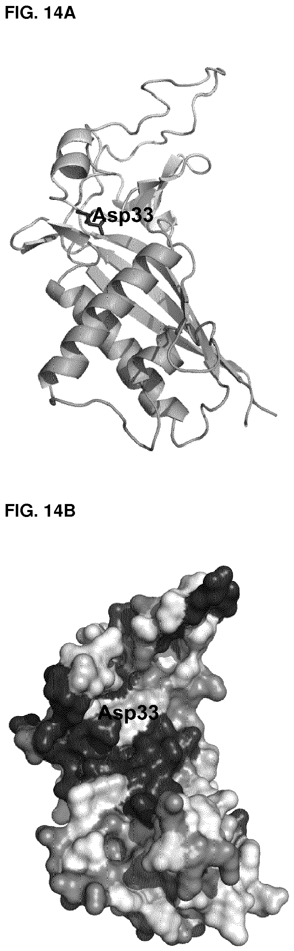

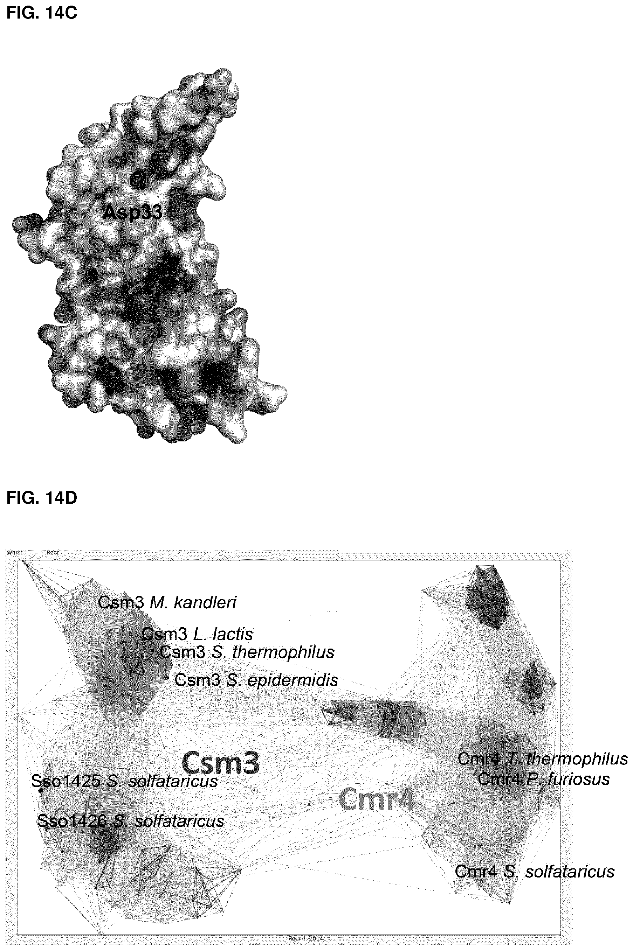

FIG. 14 shows computational analysis of Csm3. (A-C) A structural model of StCsm3 in different representations. (A) Cartoon representation with the core RRM region shown in dark and the "lid" domain shown in light. Active site residue D33 is indicated. (B) Molecular surface of the Csm3 model colored according to sequence conservation (dark - conserved, light - variable). (C) Molecular surface of the Csm3 model colored according to electrostatic potential (dark - positive, light - negative). (D) Clustering of 604 Csm3 and Cmr4 sequence homologs with CLANS. Representatives of Csm3 and Cmr4 families from experimentally characterized (Hale et al., 2009; Hatoum-Aslan et al., 2013; Hrle et al., 2013; Millen et al., 2012; Rouillon et al., 2013; Staals et al., 2013; Zhang et al., 2012) Type III CRISPR-Cas systems are labeled. Each dot represents a sequence, connecting lines represent the similarity between sequences. Thicker lines and shorter distances indicate higher sequence similarity. Only connections corresponding to P-values of le-12 or better are shown.

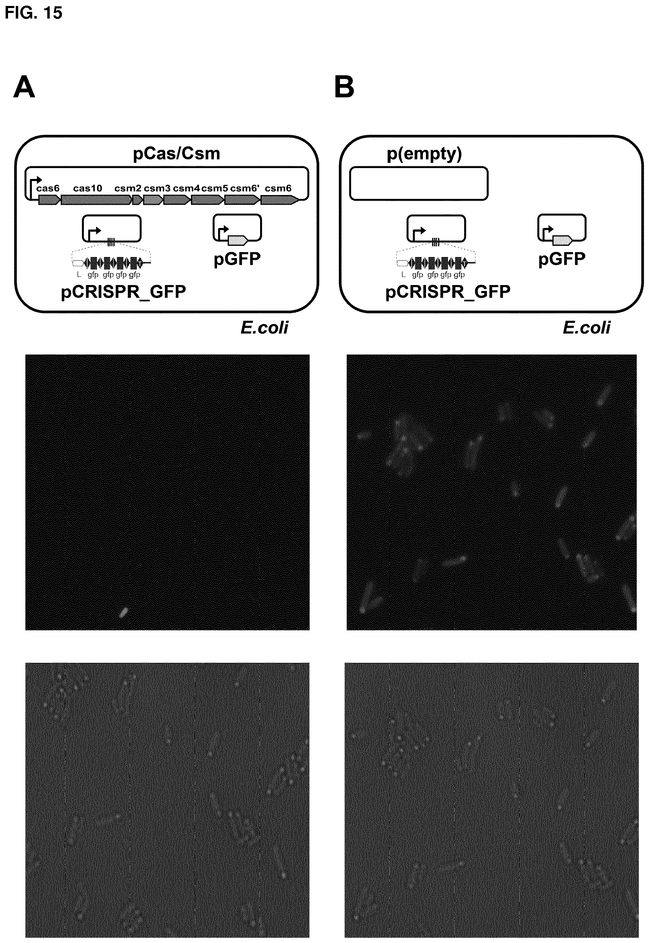

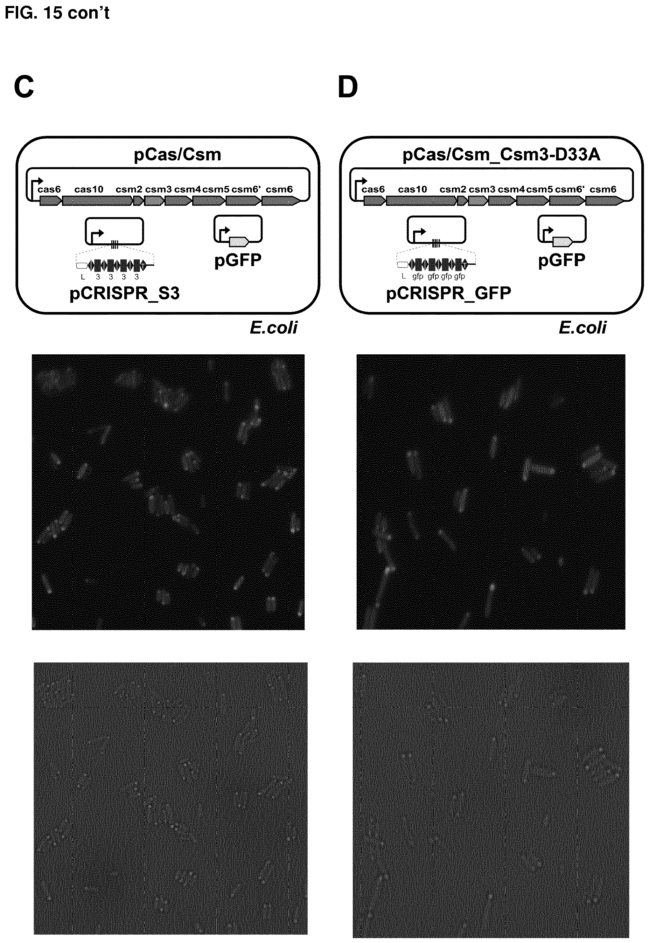

FIG. 15 shows StCsm-triggered GFP transcript degradation *in vivo. E.coli* BL21 (DE3) was transformed with three compatible plasmids (see schematic representation in panels A-D): (i) pCRISPR_MS2 plasmid bearing the synthetic CRISPR array of five repeats interspaced by four 36-nt spacers targeting the GFP transcript; (ii) pCsm/Cas plasmid for the expression of Cas/Csm proteins; (iii) pGFP plasmid for GFP expression. The StCsm and GFP transcript expression was induced in *E.coli* and the GFP transcript degradation was monitored by inspecting GFP fluorescence in the cells. The cells were imaged by contrast (see bottom images in panels A-D) and fluorescence microscopy (see top images in panels A-D).

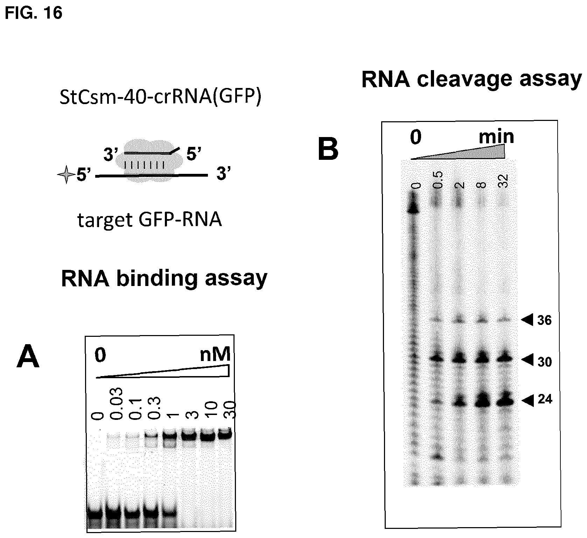

FIG. 16 shows *in vitro* cleavage activity of the StCsm targeted to the GFP transcript. Schematic representation of StCsm bound to the target RNA used for *in vitro* binding and cleavage assays is presented above the gels. RNA was 5'-end labeled with ³³P as indicated with asterisk. (A) Gel shift assay of RNA binding by StCsm-40. The binding reactions contained the ³³P-labeled S3/1 RNA (0.5 nM) and StCsm-40 at concentrations indicated by each lane. Samples were analyzed by PAGE under non-denaturating conditions. (B) StCsm-40 cleavage assay. Cleavage reactions were performed at 25°C for indicated time intervals in the Reaction buffer supplemented with 10 mM Mg-acetate, 8 nM GFP RNA substrate and 160 nM StCsm-40. Samples were analyzed by denaturing PAGE, followed by phosphorimaging. Triangles with corresponding numbers indicate cleavage product length.

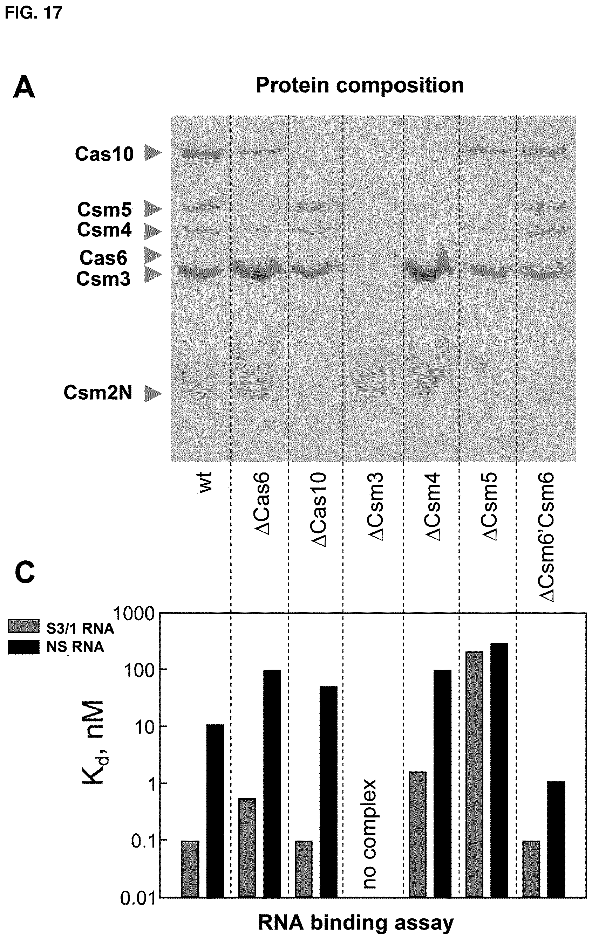

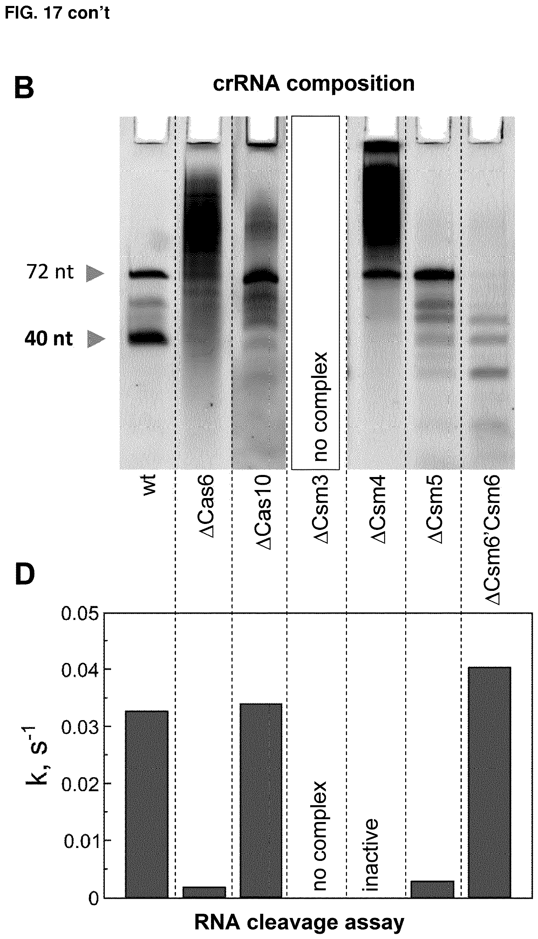

FIG. 17 shows protein composition and cleavage in vitro cleavage activity of deletion mutants of StCsm-40 complex. Single-gene deletion variants of pCas/Csm plasmid were generated by disrupting individual *cas* genes by deletions or frameshift mutations. *Escherichia coli* BL21(DE3) cells were transformed with a corresponding deletion mutant variant of pCas/Csm, pCRISPR_S3, and pCsm2-Tag plasmids. The deletion mutants of StCsm-40 complex were isolated from such cells by Strep-chelating affinity and size exclusion chromatography. (A) Protein composition of the purified StCsm-40 deletion mutant variants as revealed by SDS-PAAGE. In all cases the protein that corresponds to the disrupted *cas* gene is lacking. In case of ΔCsm4 mutant, the obtained Csm-complex also lacks Cas10, in addition to Csm4. In cells that are deprived of *csm3* gene, no complex is pull-downed by the Csm2-Tag subunit. (B) crRNAs co-purified with StCsm-complex deletion mutant variants were extracted using phenol:chloroform:isoamylalcohol and precipitated with isopropanol. Isolated nucleic acids were separated on a denaturing 15% PAAG and visualised by means of SybrGold staining. In cases of ΔCas6 and ΔCsm4, the purified nucleic acid samples contained a ribonucleic acid molecules of variable size. In almost all cases analysed (ΔCas6, ΔCas10, ΔCsm4, and ΔCsm5; with the exception of ΔCsm6'ΔCsm6) the crRNA is not fully matured 40 nt species. However, a band corresponding to 72 nt crRNA was visible in cases of ΔCas10, ΔCsm4, and ΔCsm5 mutants. (C) RNA binding affinity of StCsm complex deletion mutant variants was analyzed using electrophoretic mobility shift assay. Two ³³P-5'-labeled 68 nt RNA substrates were used for this experiment: specific S3/1 (containing a sequence fully complementary to the 36 nt crRNA encoded by spacer S3; data corresponding to it is depicted in light bars) and the non-complimentary NS RNA (dark bars). Different amounts of the StCsm (0.01-300 nM) were mixed with 0.5 nM of the RNA substrate in the binding buffer containing 1 mM EDTA. Samples were analysed using native 8% PAAG. The dissociation constants (K_{d}) for RNA binding by StCsm-40 deletion mutants were evaluated assuming the complex concentration at which half of the substrate is bound as a rough estimate of K_{d} value. Notably, while target RNA binding is significantly decreased in the case of ΔCas6 and ΔCsm4 variants, deletion of Csm5 fully abolishes target RNA binding. (D) RNA cleavage assays for StCsm-40 variants were conducted using the radioactively labelled 68 nt specific S3/1 RNA substrate. Reactions, containing 4 nM S3/1 RNA substrate and 160 nM (or 320nM) of StCsm in the reaction buffer (33 mM Tris-acetate (pH 7.9 at 25°C), 66 mM K-acetate, 0.1 mg/ml BSA, and 1 mM Mg-acetate), were initiated by addition of Mg²⁺ ions and performed at 15°C. Consequent reaction products were separated on a denaturing 20% PAGE and depicted by autoradiography. The RNA cleavage rate constants were determined by fitting single exponentials to the substrate depletion data. The obtained constants for each of StCsm variant are depicted in the graph. The cleavage activity of both ΔCas10 and ΔCsm6'ΔCsm6 are similar to wt. In all other cases the hydrolysis rate is significantly diminished. Deletion of Csm4 completely abolishes StCsm clevage activity completely.

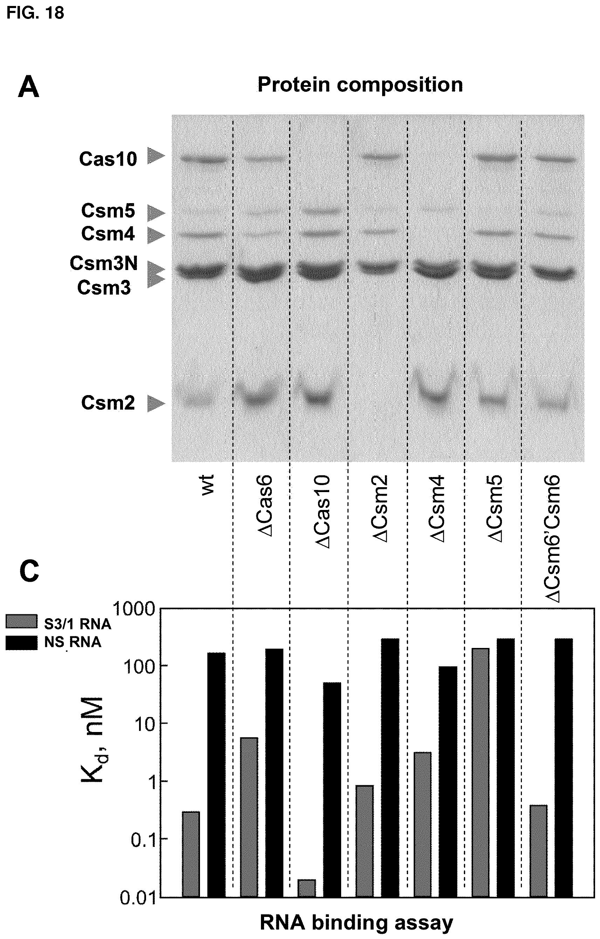

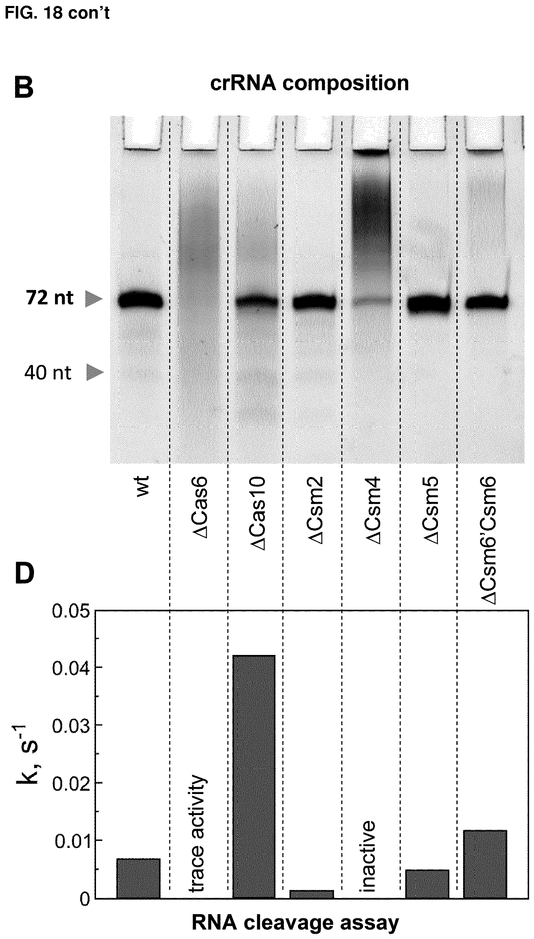

FIG. 18 shows protein composition and *in vitro* cleavage activity of deletion mutants of StCsm-72 complex. In order to obtain StCsm-72 deletion mutants, pCas/Csm deletion variants were co-expressed with pCRISPR_S3 and pCsm3-Tag plasmids in *Escherichia coli* BL21(DE3). StCsm-72 complexes were isolated by affinity and size exclusion chromatography. (A) Protein composition of the purified StCsm-72 deletion mutants as revealed by SDS-PAAGE. In all cases the protein that corresponds to the disrupted *cas* gene is lacking. ΔCsm4 variant, in addition to Csm4, lacks Cas10 subunit. (B) crRNAs that co-purify with StCsm-72 were isolated (as described in FIG. 17), separated on a denaturing 15% PAAG, and visualised by SybrGold staining. In the case of ΔCas6 and ΔCsm4 variants, the purified nucleic acid co-purified with the Csm-complex deletion variant contained a ribonucleic acid molecules of variable size. In all other cases analysed (ΔCas10, ΔCsm2, ΔCsm4, and ΔCsm6'ΔCsm6) a clear band, corresponding to 72 nt crRNA is present. (C) Electrophoretic mobility shift assay was employed to evaluate binding affinities of StCsm complexes to the complimentary target (light bars) and non-targeting (dark bars) RNAs. The experiment was performed as as described for StCsm-40 complexes (see FIG. 17). Deletion of Csm5 significantly decreased specific binding. (D) RNA cleavage assays for StCsm-72 variants were carried out similarly as desribed for StCsm-40 (see FIG. 17 legend). The graph depicts rate constants for the target RNA cleavage. The ΔCas6 or ΔCsm4 variants of StCsm-72 display almost no activity. In all other cases cleavage products, that are characteristic to StCsm-72, are visible on the gel.

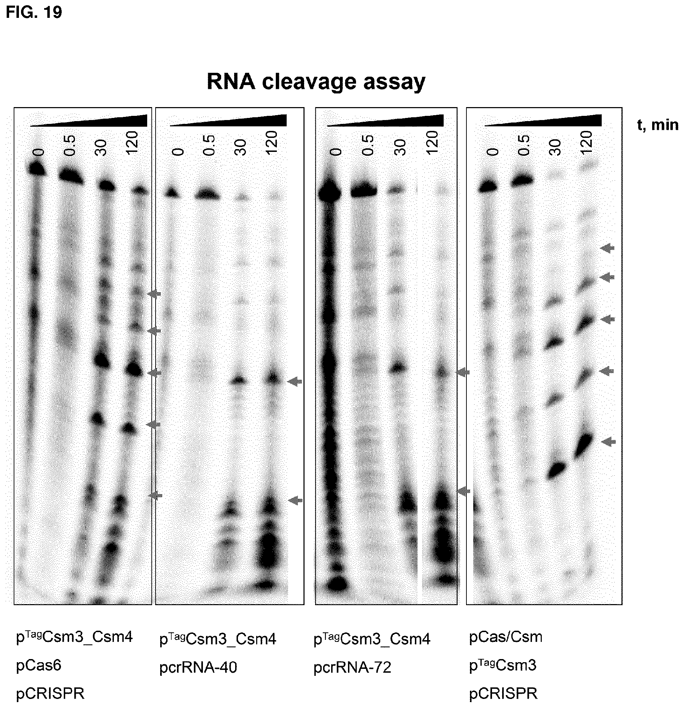

FIG. 19 shows RNA cleavage activity of the minimal StCsm complex variants. According to analysis of the StCsm deletion mutants, Csm3 and Csm4 are absolutely required for complex formation. To co-express the Csm3 and Csm4 proteins *csm3* and *csm4* genes were cloned into pCDFDuet-1 vector and Strepll-Tag sequence was fused to the N-terminal part encoding *csm3* gene to obtain p^{Tag}Csm3_Csm4. pCas6 plasmid was engineered by cloning the *cas6* gene into pCOLADuet-1 vector. The expression of Cas6 protein together with pCRISPR (encoding the S3 CRISPR array) would generate 72 nt crRNAs. Alternatively, plasmids from which 40 nt or 72 nt crRNAs could be obtained by *in vitro* transcription were constructed on the basis of pACYCDuet-1 vector (with cloned BBa J23119 promoter) and named pcrRNA-40 and pcrRNA-72, respectively. p^{Tag}Csm3_Csm4 was co-expressed with pcrRNA-40, pcrRNA-72, or both pCas6 and pCRISPR plasmids in *Escherichia coli* BL21(DE3). Subsequent Strep-chelating affinity chromatography yielded ribonucleoprotein (RNP) complexes, containing Csm3 and Csm4 proteins. The RNA cleavage activity of these RNP complexes was tested on the complimentary 68 nt S3/4 RNA target or S3/6 RNA target (containing a sequence fully complementary to the 36 nt crRNA encoded by spacer S3). Reactions, containing 4 nM RNA substrate and -15 ng/µl of the RNP complex in the reaction buffer (33 mM Tris-acetate (pH 7.9 at 25°C), 66 mM K-acetate, 0.1 mg/ml BSA, and 10 mM Mg-acetate), were initiated by addition of the purified RNP complex and incubated at 37°C for the time indicated on the top of the lanes (in minutes). Reaction products corresponding to the wt StCsm-72 complex cleavage are indicated by grey triangles. Minimal RNP complexes containing only Csm3 and Csm4 subunits and crRNA produce RNA cleavage pattern, characteristic to the wt StCsm-72 complex.

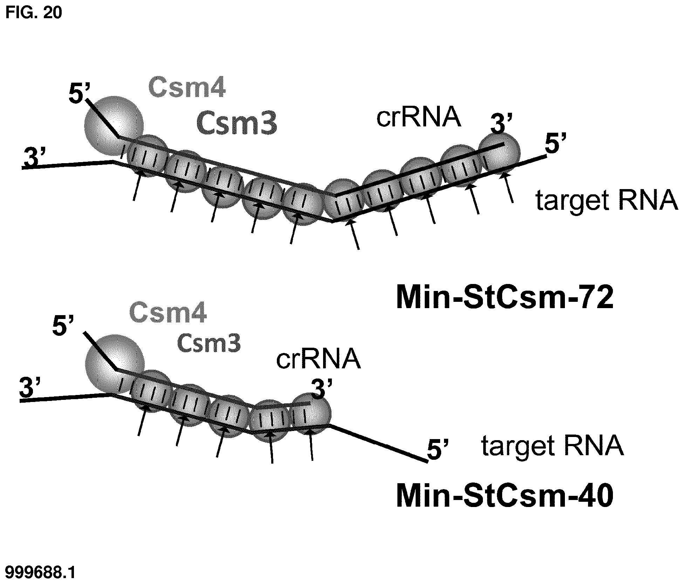

FIG. 20 shows structural and cleavage model of minimal StCsm complex variants. The minimal catalytically active StCsm complex contains Csm3 and Csm4 proteins and crRNA molecule. The 5'-handle of crRNA is recognized by the Csm4 subunit. Csm3 is endoribonuclease that cuts target RNA. The difference in the number of RNA cleavage positions suggests the different number of Csm3 subunits in the complexes.

In one embodiment, a method for the site-specific modification/shredding of a target RNA molecule in vitro is provided, by contacting under suitable conditions, a target RNA molecule and an RNA-guided RNA endonuclease complex comprising at least one RNA sequence and at least two different Csm protein subunits, to result in the target RNA molecule being modified/shredded in a region that is determined by the complimentary binding of the RNA sequence to the target RNA molecule. The method includes incubating under suitable conditions a composition that includes a target RNA molecule with a StCsm complex comprising a polyribonucleotide (crRNA) comprising a 5' handle, a 3' handle, and a spacer which is complementary, or substantially complementary, to a portion of the target RNA. In one embodiment, the crRNA lacks the 3' handle. In one embodiment, the minimal StCsm complex required for target RNA cleavage comprises Csm4 and (Csm3)ₓ (X = 1 - 10) proteins and 40 or 72 nt crRNA. In embodiments, crRNA is produced by *in vitro* transcription or chemical synthesis. In embodiments, suitable conditions means conditions *in vitro* where reaction might occur.

In embodiments, the disclosed engineered StCsm complex is used as an RNA Interference tool, to knock-out or knock-down a target RNA, such as mRNA. In one embodiment, Csm3 is modified to include a mutation. One such mutation is D33A, which inactivates the endonuclease activity of Csm3. In various embodiments, the Csm3 D33A may be used to knock-down mRNA expression. Target RNA knock-out results due to the RNA cleavage by the Csm3 protein in the Csm-complex. D33A mutation impairs target RNA cleavage but retains RNA binding ability of the Csm-complex that enables knock-down of the gene product.

StCsm complex might be isolated from a genetically modified microbe (for example *Escherichia coli* or *Streptoccocus thermophilus*). In the genetically modified microbe, components of the complex might be encoded on the one, two or three separate plasmids containing host promoters of the genetically modified microbe or promoters from a native host genome.

In one embodiment, a composition is provided, and comprising an engineered StCsm complex comprising crRNA, Csm4, and Csm3. The crRNA of the engineered complex is programmed to guide the StCsm complex to a selected site in a target RNA molecule, wherein the StCsm complex is capable of shredding the target RNA molecule under suitable conditions.

### Type III-A CRISPR-Cas loci in S. thermophilus

*S. thermophilus* strain DGCC8004 carries 13 spacers in its Type III-A CRISPR2 array (FIGS. 1A and 8). This strain also contains a Type II CRISPR1 system that is ubiquitous in the *S. thermophilus* species. In the CRISPR2 locus of DGCC8004 the 36-nt repeat sequences, that are partially palindromic, are conserved with the exception of the two terminal repeats (FIG. 1A). An A+T rich 100-bp leader sequence is located upstream of the CRISPR2 array.

DGCC8004 CRISPR2 (Type III-A) spacers range in size between 34 and 43 nt, but 36-nt spacers are the most abundant. In total, 38 unique spacers were identified among CRISPR2-positive *S. thermophilus* strains and a majority (20 out of 38) of these spacer sequences have matches (protospacers) in *S. thermophilus* DNA phage sequences, although phage interference for the *S. thermophilus* CRISPR2 locus has not yet been demonstrated. Analysis of the sequences located immediately upstream and downstream of these protospacers failed to identify any consensus sequence as a putative PAM, either due to the relatively small number of protospacers or targeting of RNA that is often PAM-independent (Hale et al., 2009). In DGCC8004, although no CRISPR2 spacer gives perfect identity with currently known sequences, 6 spacers out of 13 (S3, S4, S6, S8, S12 and S13) show strong sequence similarity with *S. thermophilus* DNA phages (at least 94% identity over at least 80% of spacer length). All phage matching protospacers appear to have been selected from the template strand. For example, the 36-nt spacer S3 matches 34 nt of a protospacer in the *S. thermophilus* phage 01205 genome (FIG. 1B). A corresponding crRNA would match the template DNA strand of the protospacer S3, and would pair with the target sequence on the coding strand of phage DNA or the respective mRNA sequence. If crRNA processing in the *S. thermophilus* Type III-A locus was similar to that in *S. epidermidis* (Hatoum-Aslan et al., 2011; Hatoum-Aslan et al., 2014; Hatoum-Aslan et al., 2013), the resulting crRNA 5'-handle in the mature crRNA would be non-complementary to the protospacer S3 3'-flank in the phage DNA coding strand or mRNA (FIG. 1B). In the *S. epidermidis* Type III-A system, which limits the spreading of plasmid DNA, the crRNA/target DNA non-complementarity outside of the spacer sequence plays a key role in silencing of invading DNA and self *vs* non-self DNA discrimination (Marraffini and Sontheimer, 2010). Taking these elements into consideration, crRNA encoded by the spacer S3 was selected as the guide, and a complementary protospacer sequence as the NA target (DNA or RNA) (FIG. 1B).

### Cloning, expression, and isolation of the S. thermophilus DGCC8004 Type III-A effector complex

To isolate the Type III-A RNP effector complex (StCsm) of the DGCC8004, the CRISPR2 locus was split into the three fragments and cloned them into three compatible vectors (FIG. 1C). Plasmid pCas/Csm contained a cassette including all the *cas*/*csm* genes (except *cas1* and *cas2*), while plasmid pCRISPR_S3 carried 4 identical tandem copies of the repeat-spacer S3 unit flanked by the leader sequence and the terminal repeat. Plasmids pCsm2-Tag or pCsm3-Tag carried a Strepll-tagged variant of *csm2* or *csm3* genes, respectively. Next, all three plasmids were co-expressed in *E. coli* BL21 (DE3) and tagged Csm2 or Csm3 proteins were isolated by subsequent Strep-chelating affinity and size exclusion chromatography.

Strep-tagged Csm2 or Csm3 proteins pulled-down from *E. coli* lysates co-purified with other Csm/Cas proteins suggesting the presence of a Csm complex (FIG. 1D). Csm complexes isolated via N-terminus Strep-tagged Csm2 (Csm2_StrepN) and the N-terminus Strep-tagged Csm3 proteins (Csm3_StrepN) were subjected to further characterization. SDS-PAGE of these complexes revealed six bands that matched the individual Cas proteins Cas6, Cas10, Csm2, Csm3, Csm4 and Csm5 (FIG. 1D). The identity of proteins in these Csm complexes was confirmed by mass spectrometry (MS) analysis (Tables 1 and 2).

The Csm complexes were examined for the presence of NA using basic phenol-chloroform extraction followed by RNase I or DNase I digestion. Denaturing PAGE analysis revealed that ∼70-nt and ∼40-nt RNA molecules co-purified with the Csm3_StrepN and Csm2_StrepN pulled-down Csm complexes, respectively (FIG. 1E). The complex isolated via Csm2_StrepN subunit also contained ∼10% of the ∼70-nt RNA. When subjected to RNase I protection assay the RNA in the complexes showed no visible degradation, indicating that the RNA is tightly bound and protected along its entire length (data not shown).

### Characterization of the crRNA

Denaturing RNA chromatography was used in conjunction with electrospray ionization mass spectrometry (ESI-MS) to analyse the crRNA sequence and determine the chemical nature of the 5'-and 3'-termini of crRNAs co-purified with both Csm complexes. Denaturing ion pair reverse phase chromatography was used to rapidly purify the crRNA directly from the Csm complexes. The crRNA isolated from the Csm3_StrepN pull-down complex revealed a single crRNA with a retention time consistent with an approximate length of 70 nt (FIG. 1F). The crRNA isolated from Csm2_StrepN pull-down complex revealed the presence of an additional crRNA, with a retention time consistent with an approximate length of 40 nt (FIG. 1G). Purified crRNAs were further analyzed using ESI-MS to obtain the accurate intact masses. A molecular weight of 22 998.5 Da was obtained for RNA isolated from Csm3 and 12 602.2 Da for RNA isolated from Csm2 pull-downs, respectively. Csm2 pull-down also contained a minor component, with a molecular weight of 12 907.3 Da (data not shown). In addition, ESI MS/MS was also used to analyze the oligoribonucleotide fragments generated from RNase A/T1 digestion of the crRNAs (FIG. 9). In conjunction with the intact mass analysis, these results revealed a 72-nt crRNA in the complex isolated via Csm3 (further termed Csm-72 according to the length of crRNA) and a 40-nt crRNA in the complex isolated via Csm2 (further termed Csm-40 complex). The MS analysis of the 72-nt crRNA is consistent with the pre-CRISPR cleavage at the base of the CRISPR RNA hairpin to yield a 8-nt 5'-handle, a 36-nt spacer and a 28-nt 3'-handle with 5'-OH and 3'-P, and could represent unmature crRNA intermediate (FIG. 1F) similar to that of Type III-A and III-B CRISPR-Cas systems (Hale et al., 2009; Hatoum-Aslan et al., 2013). Further verification of the 3'-P termini was obtained upon acid treatment of the 72-nt crRNA where no change in mass was observed using ESI-MS. Likewise, the MS analysis of the 40-nt crRNA in the Csm-40 complex revealed an 8-nt 5'-handle and a 32-nt spacer with 5'-OH and 3'-OH that would correspond to the mature crRNA (FIG. 1G). The difference in the chemical nature of the 3'-end between intermediate and mature crRNAs suggests that primary processing and final maturation are achieved by distinct catalytic mechanisms as proposed by Hatoum-Aslan for the *S. epidermidis* model system (Hatoum-Aslan et al., 2011).

### Composition and shape of the Csm complex

Evaluation of the complex composition by densitometric analysis of the SDS gels suggests the Cas10₁:Csm2₆:Csm3₁₀:Csm4₁:Csm5_{0.134} stoichiometry for Csm-72, and the Cas6_{0.10}:Cas10₁:Csm2₃:Csm3₅:Csm4₁:Csm5₁ stoichiometry for Csm-40. Fraction numbers for Cas6 and Csm5 proteins are presumably due to the weak transient interactions of these proteins in the respective complexes. Protein subunits that are involved in pre-crRNA processing, e.g. Cas6, would not necessarily occur in stoichiometric amounts in the purified effector complex.

Small angle X-ray scattering (SAXS) measurements was also performed in order to characterize the molecular mass/shape of both Csm-40 and Csm-72 effector complexes in solution. M_{w} values obtained using SAXS are in agreement both with DLS and gel-filtration data (Table 3). Taken together these data are consistent with the stoichiometry Cas10₁:Csm2₆:Csm3₁₀:Csm4₁:crRNA₁ (calculated Mw 486.2 kDa including the 72-nt crRNA) for Csm-72 and Cas10₁:Csm2₃:Csm3₅:Csm4₁:Csm5₁:crRNA₁ (calculated Mw 344.8 kDa including 40-nt crRNA) for Csm-40.

SAXS measurements revealed that the Csm-40 complex in solution has elongated and slightly twisted shape. The maximal interatomic distance (Dₘₐₓ) of the complex estimated from SAXS data is 215 Å, whereas its diameter is 75-80 Å (Table 4). The shape of this effector complex (Figure 1H) is very similar to the electron microscope structure of Cmr complexes from *Thermus thermophilus* (Staals et al., 2013), *Pyrococcus furiosus* (Spilman et al., 2013) and Cascade from *E. coli* (Wiedenheft et al., 2011) (Figure 10E). The Csm-72 complex with Dₘₐₓ of 280 Å (Table 4) is significantly more elongated than the Csm-40 complex (FIG. 1H). The lowest normalized spatial discrepancy was obtained for the end-to-end superimposition of the Csm-40 and Csm-72 models (FIG. 1H).

### Nucleic acid specificity of the Type III-A StCsm complex

In the CRISPR2 locus of DGCC8004, 34 out of 36 nt of the spacer S3 match a sequence present in the genome of *S. thermophilus* phage 01205. Thus, to probe the functional activity of the Csm-40 complex, DNA and RNA substrates were first designed that are fully complementary to the 32-nt crRNA encoded by spacer S3 and that carry phage O1205-flanking sequence. These flanking sequences lack complementarity to the 8-nt 5'-handle of the crRNA identified in the Csm-40/Csm-72 complexes (FIG. 2A and Table 5). For binding analysis DNA or RNA substrates were 5'-end radioactively labeled and the Csm-40 complex binding was evaluated by an electrophoretic mobility shift assay (EMSA) in the absence of any divalent metal (Me²⁺) ions. Csm-40 showed weak affinity for oligoduplex S3/1 DNA/DNA and DNA/RNA substrates since binding was observed only at high (100-300 nM) complex concentrations. Single-stranded S3/1 DNA (ssDNA) was bound to Csm-40 with an intermediate affinity (K_{d} ≈ 30 nM), whereas a single-stranded S3/1 RNA (ssRNA) showed high affinity binding (K_{d} ≈ 0.3 nM) (FIG. 2B). Binding competition experiments with various nucleic acids further supported the single-stranded RNA specificity for the Csm-40 complex (FIG. 2C). Cleavage data correlated with the binding affinity: S3/1 DNA/DNA, DNA/RNA and ssDNA are refractory to cleavage, whereas S3/1 ssRNA complementary to the crRNA is cut by Csm-40 in the presence of Mg²⁺ ions (FIG. 2D). RNase activity of Csm-40 complex requires Mg²⁺ or other divalent metal ions (Mn²⁺, Ca²⁺, Zn²⁺, Ni²⁺ or Cu²⁺) and is inhibited by EDTA (FIG. 11E).

Csm-40 cuts the S3/1 RNA target at 5 sites regularly spaced by 6-nt intervals to produce 48-, 42-, 36-, 30- and 24-nt products, respectively (FIGS. 2D, 2E). The sequence complementarity between the crRNA in the complex and the RNA target is a key pre-requisite for the cleavage: a non-specific RNA (FIG. 2E, bottom) was resistant to Csm-40. The Csm-40 cleavage pattern of the 3'-labeled S3/1 RNA substrate differs from that of the 5'-labeled variant. While the 5'-labeled substrate cleavage produces 48-, 42-, 36-, 30- and 24-nt products, short degradation products of 21, 27, and 33 nt (1 nt shift is due to an additional nucleotide added during the 3'-labeling) are visible on the gel (FIGS. 2D, 2E). Taken together, cleavage data for the 5'- and 3'-end labeled RNA substrates suggest that Csm-40 cuts the RNA molecule initially at its 3'-end and endonucleolytic degradation is further extended towards the 5'-end with 6-nt increments.

The Csm-72 complex carrying a 72-nt crRNA (8-nt 5'-handle plus 36 nt of the spacer S3 and 28 nt of the 3'-handle, FIG. 11A) showed ∼ 30-fold weaker binding affinity (K_{d} ≈ 10 nM) to S3/1 RNA in comparison to the Csm-40 (FIG. 11B). Nevertheless, similar to the Csm-40 complex, in the presence of Mg²⁺ ions Csm-72 cleaved S3/1 RNA, albeit at a decreased rate which may correlate with its weaker binding affinity (FIG. 11C). The 5'- and 3'-labeled S3/1 RNA cleavage pattern is identical to that of Csm-40 (FIGS. 11C, 11D and data not shown). Like the Csm-40 complex, Csm-72 showed no cleavage of S3/1 ssDNA, DNA/DNA or DNA/RNA substrates (data not shown). The heterogeneous Csm complex isolated from the *E. coli* host carrying the wt CRISPR array containing 13 spacers produces RNA cleavage products identical to those of the homogenous StCsm (FIG. 11F). Taken together, these data unambiguously demonstrate that Csm-40 and Csm-72 complexes *in vitro* target RNA but not DNA, and cut RNA at multiple sites regularly spaced by 6-nt intervals.

### Reprogramming of the StCsm complex

To demonstrate that the Type III-A StCsm complex can be reprogrammed to cut a desired RNA sequence *in vitro,* we designed and isolated Csm complexes loaded with crRNA(+Tc) or crRNA(-Tc) targeting, respectively, the 68-nt sense(+) and anti-sense(-) mRNA fragments obtained by *in vitro* transcription of the tetracycline (Tc) resistance gene in the pBR322 plasmid (nts 851-886) (FIG. 12A and Table 5). Both Csm-40 and Csm-72 complexes guided by the crRNA(+Tc) sliced the complementary sense RNA fragment but not the antisense RNA sequence (FIG. 12B). In contrast, Csm-40 and Csm-72 complexes guided by the crRNA(-Tc) cleaved antisense RNA but not a sense Tc mRNA fragment (FIG. 12B). In both cases target RNA was cleaved at multiple sites regularly spaced by 6-nt intervals (FIG. 12C).

To demonstrate that StCsm complex can be reprogrammed to cut the desired RNA target and silence gene expression *in vivo,* we designed Csm complexes targeting the GFP gene transcript in the heterologous *E.coli* host. *E.coli* BL21 (DE3) was transformed with three compatible plasmids: (i) pCRISPR_MS2 plasmid bearing the synthetic CRISPR array of five repeats interspaced by four 36-nt spacers targeting the GFP transcript; (ii) pCsm/Cas plasmid for the expression of Cas/Csm proteins; (iii) pGFP plasmid for the GFP expression, and the GFP transcript degradation was monitored by inspecting GFP fluorescence in *E.coli* cells (FIG. 15). No GFP fluorescence is detected when wt StCsm targeting the GFP transcript is expressed in *E.coli* (FIG. 15A). On the other hand, GFP fluorescence is observed in *E.coli* cells lacking the StCsm complex (FIG. 15B), or bearing the RNA-cleavage deficient Csm3-D33A mutant complex (FIG. 15C), or containing StCsm complex with CRISPR2 S3 crRNA (FIG. 15D). In a separate set of the experiments we show that isolated StCsm-40 loaded with GFP mRNA targeting crRNA (GFP) specifically binds and cuts 68 nt length GFP RNA *in vitro* (FIG. 16).

### Target RNA determinants for cleavage by crRNA-guided Csm complex

Whether the nucleotide context downstream or upstream of the protospacer sequence modulates RNA cleavage by the Csm complexes was further examined. To this end, the S3/2 RNA substrate was designed in which the flanking regions originating from 01205 phage DNA in the S3/1 substrate are replaced by different nucleotide stretches that are non-complementary to the 5'-handle of crRNA in the Csm-40 and Csm-72 complexes, and to the 3'-handle in the Csm-72 complex. RNA binding and cleavage data showed that despite differences in the nucleotide context of flanking sequences in the S3/1 and S3/2 substrates, cleavage patterns for the Csm-40 and Csm-72 complexes are nearly identical, except for an extra 18-nt product for the Csm-72 (FIG. 11C).

Whether the base-pairing between the flanking sequences of the RNA target and 5'- and 3-handles of crRNA in the Csm-40 and Csm-72 complexes affect either the cleavage efficiency or pattern was examined. We designed S3/3, S3/4, and S3/5 RNA substrates that contain flanking sequences complementary to the 5'-handle (40- or 72-nt crRNA), 3'-handle (72-nt crRNA) or both 5'- and 3'-handles in 72 nt-crRNA, respectively (FIG. 13A and Table 5). The cleavage analysis revealed that base-pairing between the 8-nt 5'-handle of crRNA and the 3'-flanking sequence had no effect on the cleavage pattern of the Csm-40 and Csm-72 complexes. The S3/3 substrate is cleaved with the same 6-nt step by Csm-40, suggesting that the non-complementarity of the flanking sequences is not a necessary pre-requisite for cleavage by the Csm complex (FIG. 13). For the Csm-72 complex, extension of the base-pairing between the 3'-handle of the 72-nt crRNA and the protospacer 5'-flanking sequence in S3/5 RNA substrate results in target RNA cleavage outside the protospacer, yielding 12- and 6-nt cleavage products (FIG. 3). Moreover, the S3/6 substrate, which has extended complementarity between crRNA 3'-handle and 5'-flanking sequence was cleaved at multiple positions along the full length of RNA duplex, except for the region complementary to the crRNA 5'-handle (FIG. 3). The cleavage at 18 and 12 nt outside the protospacer was also detected for the Csm-40 complex on S3/4 and S3/5 RNA substrates (FIG. 13). The 40-nt crRNA present in the Csm-40 complex lacks the 3'-handle and therefore cannot form RNA duplex with the 5'-flanking sequence in the S3/5 and S3/6 RNA substrates. However, the Csm-40 complex preparation still contains ∼ 10% of unmatured 72-nt crRNA, and this heterogeneity results in the extra cleavage outside the protospacer (FIG. 13C).

To interrogate the importance of base-pairing within the protospacer region for target RNA cleavage, a set of RNA substrates was designed harboring two adjacent nucleotide mutations in the spacer region (substrates S3/7, S3/8 and S3/9, see FIG. 13A and Table 5). Two nucleotide mismatches in these substrates did not compromise RNA cleavage by the Csm-40 (FIG. 6) and Csm-72 complexes (data not shown), suggesting that the StCsm complex tolerates at least two contiguous mismatches in the protospacer region homologous to the crRNA.

To explore whether 3'- or 5'-ends of the target RNA are important for cleavage by the Csm-40 complex, a set of truncated RNA substrates was designed. In S3/10, S3/14 and S3/12 RNA substrates unpaired flaps at the 3'-, 5'- or both ends of the target RNA were truncated, while in S3/11 and S3/13 substrates the truncations extend into the region complementary to crRNA (FIG. 4A). Binding affinity for most of the truncated substrates was not compromised (FIG. 4B) and target RNA cleavage occurred at multiple sites spaced by 6-nt intervals at conserved protospacer positions (FIG. 4C). Truncations extending into the protospacer region (S3/11 and S3/13) showed decreased binding and reduced cleavage rates. This could be a result of the decreased duplex stability; however, the role of the "seed" sequence cannot be excluded. For all RNA substrates the cleavage sites were located at a fixed distance with respect to the conserved 5'-handle of crRNA (FIG. 4C).

### Identification of the ribonuclease subunit in the StCsm complex

Regularly spaced cleavage pattern of the RNA target (FIGS. 2-4, 11-13) implies the presence of multiple cleavage modules in the Csm complex. According to the densitometric analysis, 3 Csm2 and 5 Csm3 subunits are identified in the Csm-40 complex, while 6 Csm2 and 10 Csm3 subunits are present in the Csm-72 complex. Multiple copies of the Csm2 and Csm3 proteins in the Csm complexes make them prime candidates for catalytic subunits. StCsm2 is a small (121 aa) α-helical protein of unknown structure. StCsm3 (220 aa) contain a conserved RRM core and is fairly closely related (∼35% sequence identity) to *Methanopyrus kandleri* Csm3, whose crystal structure has been solved recently (Hrle et al., 2013). It was reasoned that, since the catalytic activity of the StCsm complex requires the presence of Me²⁺ ions, the active site is likely to contain one or more acidic residues. Multiple sequence alignments of both Csm2 and Csm3 protein families were inspected for conserved aspartic or glutamic residues. No promising candidates in StCsm2 were found but several, including D33, D100, E119, E123, and E139 were identified in StCsm3 (FIG. 5A). To probe the role of these conserved negatively charged Csm3 residues, single residue alanine replacement mutants were constructed. The H19A mutant was also constructed, since it was shown that the corresponding mutation (R21A) in *M. kandleri* Csm3 abolished binding of single-stranded RNA (Hrle et al., 2013). Each mutant was expressed in the context of other StCsm/Cas proteins and analyzed the cleavage activity of the StCsm-40 complex containing mutant Csm3 subunits. StCsm3 H19A, D100A, E119A, E123A, and E139A mutants did not compromise the formation, RNA binding or cleavage activity of Csm-40 complex (FIG. 5B-5F). However, the D33A mutant impaired Csm-40 RNA cleavage (FIG. 5E-5F) without affecting RNA binding (FIG. 5D) or complex assembly. Taken together, these data demonstrate that Csm3 is an RNase, producing multiple cleavage patterns spaced by regular 6-nt intervals, and that the D33 residue is part of the catalytic/metal-chelating site. StCsm3 structural model based on the homologous structure of *M. kandleri* Csm3 is in good agreement with the identified role for this residue (FIG. 14A). D33 belongs to the highly conserved surface patch that extends from the RRM core into the "lid" subdomain (FIG. 14B). Part of this surface patch is positively charged, supporting the idea that it represents an RNA-binding site (FIG. 14C).

### In vivo RNA targeting by the StCsm complex

To test whether the StCsm complex can target RNA *in vivo,* the MS2 phage restriction assay was employed. MS2 is a lytic single-stranded RNA coliphage which infects *E. coli* via the fertility (F) pilus. The MS2 phage is a preferable model to investigate RNA targeting by the CRISPR-Cas system *in vivo* as no DNA intermediate is formed during the life cycle of this phage (Olsthoorn and van Duin, 2011). For *in vivo* RNA-targeting experiment, the *E.coli* NovaBlue (DE3, F⁺) strain was transformed with two compatible plasmids: i) pCRISPR_MS2 plasmid bearing the synthetic CRISPR array of five repeats interspaced by four 36-nt spacers targeting correspondingly the mat, lys, cp, and rep MS2 RNA sequences, and ii) pCsm/Cas plasmid for the expression of Cas/Csm proteins (FIG. 6A). The phage- targeting and control *E. coli* strains were plated and infected with series of dilutions of MS2 using the drop plaque assay. The assay revealed that the *E. coli* strain expressing wt Csm and crRNAs that target MS2 induces a 3 to 4 Log reduction of the plaquing efficiency with respect to the control cells (FIG. 6). No resistance to the MS2 phage infection was observed in the strain expressing either the non-targeting crRNA or the cleavage-deficient (D33A) Csm3 mutant. Taken together these data demonstrate that the StCsm complex conveys *in vivo* resistance to RNA phage in the heterologous *E. coli* host.

We established the NA specificity and mechanism for the Type III-A CRISPR-Cas system of *Streptococcus thermophilus.* In sharp contrast to other CRISPR-Cas subtypes, the functional activity of Type III-A system so far has not been reconstituted *in vitro.* Cas/Csm proteins in the Type III-A CRISPR locus of the *S. thermophilus* DGCC8004 are homologous to those of *S. thermophilus* DGCC7710 and LMD-9. They also show more distant but significant similarities to Cas/Csm proteins of *L. lactis, E. italicus* and S. *epidermidis* (Marraffini and Sontheimer, 2008; Millen et al., 2012) (FIG. 8).

### Csm complexes of S. thermophilus

The Type III-A CRISPR-Cas locus of the DGCC8004 was expressed in *E. coli* and two RNP complexes, termed Csm-40 and Csm-72, were isolated. Both complexes share a conserved set of Cas10, Csm2, Csm3 and Csm4 proteins. In addition to this core, the Csm-40 also contains the Csm5 protein. Two distinct crRNAs of 72- and 40-nt co-purify with Csm-40 and Csm-72 complexes isolated from the heterologous *E. coli* host. The 72-nt crRNA comprised of an 8-nt 5'-handle, a 36-nt spacer and a 28-nt 3'-handle would result from the pre-crRNA cleavage between 28 and 29 nt within the conserved repeat region presumably by the Cas6 nuclease, similar to the III-B CRISPR-Cas system (Carte et al., 2008). The shorter 40-nt crRNA co-purified with the Csm-40 complex of *S. thermophilus* contains the conserved 8-nt 5'-handle and 32-nt spacer indicating that the 72-nt crRNA intermediate undergoes further 3'-end processing to produce a mature 40-nt crRNA that lacks the 3'-handle and 4 nt within the spacer region (FIG. 7). The RNase involved in the maturation of 72 nt crRNA intermediate remains to be identified, however the Csm5 protein which is absent in Csm-72 but is present in Csm-40 could be a possible candidate. Indeed, *csm5* gene deletion in DGCC8004 produces only unmatured Csm-72 complexes (data not shown).

The crRNA processing and maturation pathway in the *S. thermophilus* Type III-A system (FIG. 7) shows striking similarity to that in *S. epidermidis.* First, the SeCsm complex includes the same set of Cas10, Csm2, Csm3, Csm4 and Csm5 proteins as the StCsm-40. Furthermore, in *S. epidermidis,* the primary processing by Cas6 produces a 71-nt crRNA intermediate, that is subjected to further endonucleolytic processing at the 3' end (Hatoum-Aslan et al., 2011; Hatoum-Aslan et al., 2014).

### StCsm complex cuts RNA producing a regular cleavage pattern

The Csm complexes of *S. epidermidis* and *S. solfataricus* have been reconstituted and isolated, however the NA cleavage activity has not been reported so far. *In vivo* studies in *S. epidermidis* suggested that the Type III-A SeCsm RNP complex targets DNA (Marraffini and Sontheimer, 2008) in a PAM-independent manner and prevents autoimmunity by checking the complementarity between the crRNA 5'-handle and the 3'-flanking sequence in the vicinity of the protospacer (Marraffini and Sontheimer, 2010). In contrast to these data we found that the StCsm-40 and StCsm-72 complexes bind ssRNA with high affinity and cut a ssRNA target in a PAM-independent manner in the presence of Me²⁺ ions, producing a regular 6-nt cleavage pattern in the protospacer region (FIGS. 2D and 11C-11E). In this respect the Type III-A StCsm complex resembles the RNA-targeting Type III-B Cmr-complexes PfCmr, SsCmr and TtCmr (Hale et al., 2009; Staals et al., 2013; Zhang et al., 2012) (FIG. 7) rather than DNA targeting Type I and II complexes. By targeting RNA rather than DNA, the StCsm complex avoids autoimmunity. It was demonstrated that the nucleotide context and non-complementarity outside the protospacer have no effect on the target RNA cleavage, demonstrating that PAM or unpaired flanking sequences of the protospacer are not required for cleavage by the StCsm (FIG. 13). The complementarity of the protospacer is the only pre-requisite for the StCsm cleavage: non-matching RNA is not cleaved; however, either two contiguous mismatches or end truncations in the complimentary protospacer S3 are tolerated (FIG. 13). The differences in the cleavage patterns of the 5'- and 3'-labeled RNAs (FIG. 2D) imply that cleavage first occurs at 3'-end of the target RNA. It remains to be established whether the observed cleavage pattern is dictated by the "seed" sequence (eg. directionality of base pairing process between the crRNA and target RNA) or by nucleotide context-dependent differences of cleavage rate.

It was found that for the Csm-72 complex the target RNA is being cleaved at regular 6-nt intervals outside the protospacer if it retains base complementarity to the crRNA 3'-handle. Such regularly spaced cleavage pattern of the RNA target (FIGS. 2-4, 11 and 13) implies the presence of multiple cleavage modules in the Csm complex. The major difference between the Csm-40 and Csm-72 complexes is the number of Csm2 and Csm3 subunits. The Csm-40 contains 3 Csm2 and 5 Csm3 subunits while Csm-72 contains 6 Csm2 and 10 Csm3 subunits (FIG. 1D). The size of the complexes determined by SAXS correlates with the different stoichiometry of Csm-40 and Csm-72. Both complexes show a slightly twisted elongated shape but the Csm-72 is significantly more elongated than Csm-40 complex (FIG. 1H). Taken together these data suggest that the longer unmatured 72-nt crRNA intermediate in the Csm-72 complex binds additional copies of Csm2 and Csm3 subunits into a RNP filament (FIG. 7).

### Csm3 is a RNase subunit in the StCsm complex

Computational analysis revealed that StCsm3 has a conserved RRM core and is fairly closely related (∼35% sequence identity) to *M. kandleri* Csm3 (Hrle et al., 2013). StCsm3 displays close structural similarity to MkCsm3, in particular the RRM-core and insertions into RRM-core that form the "lid" subdomain (FIG. 14A). In contrast, StCsm3 lacks both the N-terminal zinc binding domain and the C-terminal helical domain, making its structure more compact compared to that of MkCsm3. Thus, StCsm3 may be considered as a trimmed-down version of MkCsm3. Guided by the multiple sequence alignment and homology model of StCsm3, candidate active site/metal chelating residues of Csm3 were selected and subjected to alanine mutagenesis. The highly conserved D33 residue of the StCsm3 was critical for the RNA cleavage activity of the Csm complex, demonstrating that Csm3 is an RNase in the StCsm and other Type III-A CRISPR-Cas systems (FIG. 5).

### Implications for other RNA-targeting CRISPR systems

The StCsm complex was specific for RNA and cuts it in a PAM-independent manner producing a regular 6-nt cleavage pattern. The Csm3 protein, which is present in Csm-40 and Csm-72 complexes in multiple copies, was demonstrated to act as an RNase responsible for the target RNA cleavage. In this respect the Type III-A Csm complex of *S. thermophilus* closely resembles the RNA targeting Type III-B Cmr complex of *T. thermophilus* (TtCmr complex) that also produces a regular 6-nt cleavage pattern (Staals et al., 2013). The RNA degrading subunit in the Type III-B Cmr-module remains to be identified. Although there is currently no experimental evidence, Staals et al. suggested that Cmr4 could fulfill this role (Staals et al., 2013). Clustering of Csm3 and Cmr4 homologs by sequence similarity revealed that they form two related but separate groups (FIG. 14D). On the other hand, neither Csm3 nor Cmr4 families are homogenous. They are comprised of sequence clusters of various sizes. StCsm3 is a member of a large representative group of Csm3 homologs that includes those from *S. epidermidis, L. lactis* and *M. kandleri.* Another large, but more loosely connected group does not have proteins from experimentally characterized systems, except for the Csm complex from *S. solfataricus.* Sso1425 and Sso1426, two of its Csm3-like proteins (Makarova et al., 2011a), are members of this group albeit they are non-typical. The Cmr4 family appears even more heterogeneous than Csm3. Cmr4 proteins of experimentally characterized III-B systems from *T. thermophilus* and *P. furiosus* represent one of the larger clusters, while Cmr4 from *S. solfataricus* is a non-typical outlier. Biochemical characterization revealed that PfCmr and TtCmr RNA cleavage mechanism are similar and follow a 3'- or 5-' ruler mechanism, respectively (Hale et al., 2009; Staals et al., 2013). Meanwhile, SsCmr endonucleolytically cleaves both target RNA and crRNA at UA dinucleotides (Zhang et al., 2012). It thus would not be surprising if members of other, so far experimentally uncharacterized groups were part of Cmr complexes with somewhat different properties.

We questioned if Csm3 and Cmr4 proteins may have similarly organized active site. The aligned sequences of Csm3 and Cmr4 subunits from characterized systems revealed that sequences of both families have Asp in the corresponding positions, suggesting similar active sites (FIG. 5A). The exception is Sso1426. This is surprising, considering the composition of the *S. solfataricus* Csm complex. Four copies of Sso1426 were found to be present within the complex suggesting that this subunit might play a role of the Csm3 (Rouillon et al., 2013). In contrast, another Csm3-like protein Sso1425 does have the D33 counterpart suggesting it can cleave ssRNA. However, only a single copy of Sso1425 was found in the *S. solfataricus* complex. Taken together, these data suggest that Csm-modules in *S. thermophilus* and *S. solfataricus* have different architectures and RNA cleavage mechanisms.

It is demonstrated for the first time that the Csm effector complex of the *S. thermophilus* Type III-A system targets RNA and establish the mechanism of RNA cleavage. It is demonstrated that in the Type III-A effector complex Cas/Csm proteins assemble into an RNP filament (FIG. 7) that contains multiple copies of Csm2 and Csm3 proteins. The inventors provided evidence that the Csm3 subunit acts as an RNase that cleaves target RNA at multiple sites spaced by regular 6-nt intervals (FIG. 7). The number of cleavage sites correlates with the number of Csm3 subunits in the Csm effector complex. Easy programmability of the Type III-A StCsm complex by custom crRNAs (FIG. 12), paves the way for the development of novel molecular tools for RNA interference.

RNA cleavage specificity established here for the StCsm complex *in vitro* is supported by *in vivo* experiments of MS2 RNA phage interference in the heterologous *E.coli* host (FIG. 6). It remains to be established whether RNA silencing by the StCsm complex can contribute to the DNA phage interference in the *S. thermophilus* host. Transcription-dependent DNA targeting mechanism has been proposed recently for the Type III-B CRISPR-Cmr system (Deng et al., 2013); however, it has yet to be demonstrated for *S. thermophilus* and other Type III-A systems.

### Deletion analysis of StCsm complex

Csm-complexes are composed of several Cas proteins (Cas10, Csm2, Csm3, Csm4, Csm5) and contain traces of Cas6. In the StCsm complexes Csm3 acts as the ribonuclease that cuts target RNA. Cas6 is responsible for the pre-crRNA maturation into 72 nt crRNAs. To establish a functional role of other Csm proteins, we engineered pCas/Csm plasmid variants with disrupted individual *cas*/*csm* genes and isolated a set of StCsm deletion mutant complexes. These StCsm deletion mutant complexes were then subjected to biochemical analyses to determine the role of each individual protein in the StCsm complex assembly and RNA cleavage.

First we examined the composition of proteins and crRNAs in the StCsm complex deletion mutants. SDS-PAGE analysis of protein composition of the purified StCsm-40 and StCsm-72 deletion mutants confirmed that in all cases protein corresponding to the disrupted *cas* gene is missing in the complex (FIG. 17A, FIG. 18A). Csm3 makes a backbone of StCsm complex since no complex is detected when *csm3* gene is deleted. Cas10 seems to be associated to the Csm4 in the Csm-complex since Cas10 subunit is missing in the StCsm-40ΔCsm4 and StCsm-72ΔCsm4 samples.

crRNAs co-purified with deletion mutant complexes are distinct (FIG. 17B, FIG. 18B). Nucleic acids purified from StCsm-40ΔCas6 and StCsm-72ΔCas6 complexes pre-dominantly contain long pre-crRNAs, that support Cas6 function in crRNA maturation. In the case of StCsmΔCsm4 variant 72 nt crRNA co-purifies together with long pre-crRNA molecules implying that crRNA binding specificity is compromised. Wt StCsm-40 and StCsm-40ΔCsm6'ΔCsm6 predominantly contains 40 nt crRNAs, while in all other cases 72 nt prevails in the Csm-complex. Taken together, available data suggest that Cas10, Csm5 and possibly Csm2 and Csm4 proteins are important for crRNA maturation from 72 to 40 nt species.

Next we explored the impact of single protein deletions on the StCsm complex capability to bind and cleave RNA. StCsm complex deletion mutants were probed on target substrates complementary to the crRNA encoded by the spacer S3 and non-targeting RNA substrates (FIG. 17C, FIG. 18C). Csm5 deletion dramatically impacts specific RNA binding: StCsmΔCsm5 complexes bind target and non-target RNAs with nearly the same affinity while wt StCsm complexes show ∼100-fold tighter binding to the target RNA. To compare the RNA cleavage capabilities of the StCsm complex variants, we performed cleavage assays and determined cleavage rate constants (FIG. 17D, FIG. 18D). Surprisingly, most of the StCsm complex deletion mutants retained at least partial RNA cleavage activity. Only for StCsmΔCas6 and StCsmΔCsm4 complexes RNA cleavage activity was nearly fully abolished. In all other cases, the reaction products (and hence cleavage positions) were identical. The cleavage activity of StCsmΔCsm6'ΔCsm6 variants is similar to that of wt. This is not surprising since Csm6' and Csm6 proteins are not present in the StCsm complex. Cas10 significantly impacted only the yield of the complex but had no effect on the cleavage rate. The RNA cleavage assay data suggests that only Csm3, Csm4, and crRNA-generating Cas6 are required for the target RNA cleavage. Csm2 is completely dispensable in respect to RNA cleavage.

### Minimal StCsm complex assembly

Deletion analysis shows that Csm3 and Csm4 proteins are critical for Csm-complex assembly/activity. Therefore, we explored the possibility to assemble a minimal Csm-complex arranged of three components including Csm3 and Csm4 subunits, and crRNA. Such minimal engineered variant StCsm would be a convenient tool for specific RNA targeting both *in vitro* and *in vivo.* We cloned the *csm3* and *csm4* genes into pCDFDuet-1 vector and added a Strepll-Tag sequence to the N-terminal part of *csm3* to obtain p^{Tag}Csm3_Csm4 plasmid. pCas6 plasmid was constructed by cloning *cas6* gene into pCOLADuet-1 vector. The expression of Cas6 protein together with pCRISPR_S3 (encoding the S3 CRISPR region) would generate unmatured 72-nt crRNA which would be incorporated in Csm ribonucleoprotein complex. Alternatively, to omit Cas6-mediated pre-crRNA maturation, plasmids pcrRNA-40 and pcrRNA-72 were constructed. Transcription of these plasmids in *E.coli* will produce 40-nt or 72-nt crRNA species in the absence of Cas6. These plasmids were engineered on basis of pACYCDuet-1 vector with under a control of the BBa J23119 promoter. p^{Tag}Csm3_Csm4 was co-expressed in *E.coli* BL21(DE3) either with pCas6 and pCRISPR plasmids or with pcrRNA-40/pcrRNA-72 plasmids. Affinity purification on the Strep-chelating column yielded minimal Csm complexes, containing Csm3 and Csm4 protein subunits and crRNA. When pCas6 plasmid was present in the expression system, Cas6 protein co-purified with the Csm-complex. The RNA cleavage activity of these RNP complexes was assayed on the 68-nt S3/4 or 86-nt S3/6 RNA target substrate (FIG. 19). Minimal Csm-complexes containing only Csm3 and Csm4 subunits, show RNA cleavage pattern, characteristic to the wt StCsm-72 complex. Taken together, data provided here show that minimal Csm complex assembled using only Csm3, Csm4, and crRNA cleaves target RNA. In this respect, it provides a versatile tool for RNA knock-outs in the cell. Cleavage deficient variant of the minimal complex could be used for RNA knock-downs or pull-down of the desired target RNA from cells.

### Experimental procedures

### Expression and isolation of Csm complexes

The sequence of CRISPR2-cas locus of *S. thermophilus* DGCC8004 was deposited in GenBank (accession number KM222358). Heterologous *E. coli* BL21(DE3) cells producing the Strep-tagged Csm complexes were engineered and cultivated as described. Csm-40 and Csm-72 complexes were isolated by subsequent Strep-chelating affinity and size exclusion chromatography steps.

*Streptococcus thermophilus* DGCC8004 was cultivated at 42°C in MI 7 broth (Oxoid) supplemented with 0.5% (w/v) lactose. Chromosomal DNA was extracted and purified using GeneJET Genomic DNA Purification Kit (Thermo Scientific). CRISPR2-Cas region was amplified by polymerase chain reaction (PCR) and sequenced using primers designed by genomic comparison with S. *thermophilus* DGCC7710 (GenBank accession number AWVZ01000003). Annotation of the predicted ORFs was performed using BLASTP at NCBI (http://blast.ncbi.nlm.nih.gov/Blast.cgi). CRISPR region was identified through repeat sequence similarity to that of *S. thermophilus* DGCC7710. Multiple sequence alignments of *cas*/*csm* genes, spacers and repeats sequences were carried out with ClustalW2 (http://www.ebi.ac.uk). Genomic DNA isolated from *S. thermophilus* DGCC8004 strain was used as the template for PCR amplification of the *cas*/*csm* genes. DNA fragment covering the 8.5 kb *cas6-cas10-csm2-csm3-csm4-csm5-csm6-csm6'* gene cassette was cloned into pCDFDuet-1 expression vector via Ncol and AvrII restriction sites in two separate subcloning steps to generate plasmid pCas/Csm. Individual *cas*/*csm* genes were cloned into pETDuet-1_N-Strepll and pETDuet-1_C-Strepll expression vectors, except of *cas10* (which was cloned into pBAD24 C-His-StrepII-His) and *csm6* or *csm6'* (that were cloned into pBAD24 N-His-Strepll-His) to generate pCsmX-Tag and pCasY-Tag plasmids, where X=2,3,4,5,6,6' and Y=6,10. A synthetic 445-nt CRISPR locus containing five 36-nt length repeats interspaced by four identical 36-nt spacers S3 of the *S. thermophilus* DGCC8004 CRISPR2 system was obtained from Invitrogen and cloned into the pACYC-Duet-1 vector to generate a plasmid pCRISPR S3. Four copies of the spacer S3 have been engineered into the pCRISPR_S3 plasmid to increase the yield of the Csm-crRNA complex. Full sequencing of cloned DNA fragments confirmed their identity to the original sequences.

All three plasmids were co-expressed in *Escherichia coli* BL21 (DE3) grown at 37°C in LB medium supplemented with streptomycin (25 ng/µ1), ampicilin (50 µg/µl), and chloramphenicol (30 µg/µ1). The fresh LB medium was inoculated with an overnight culture (1/20 (v/v)), and bacteria were grown to the mid-log phase (0D₆₀₀ₙₘ 0.5 to 0.7), then 1 mM IPTG (and 0.2% (w/v) L-(+)-arabinose in case of Cast 0, Csm6 and Csm6') was added and cell suspension was further cultured for another 4 h. Harvested cells were resuspended in a Chromatography buffer (20 mM Tris-HC1 (pH 8.5), 0.5 M NaC1, 7 mM 2-mercaptoethanol, 1 mM EDTA) supplemented with 0.1 mM phenylmethylsulfonyl fluoride (PMSF), and disrupted by sonication. Cell debris was removed by centrifugation. Csm complexes were captured on the StrepTrap affinity column (GE Healthcare) and further subjected to the Superdex 200 size exclusion chromatography (prep grade XK 16/60; GE Healthcare). SDS-PAGE of individual Strep-tagged Csm2, Csm3, Csm4, CsmS, Cas6 and Cas10 proteins isolated by affinity chromatography from E. coli lysates revealed co-purification of other Csm/Cas proteins suggesting the presence of a Csm complex. The abundance of the Csm complex co-purified via the Csm4-, CsmS-, Cas6- and Cas10-Strep tagged subunits was very low, and no complex was pull-downed via Csm6 or Csm6' subunits (data not shown). Therefore, Csm complexes isolated via N-terminus Strep-tagged Csm2 (Csm2 StrepN) and the N-terminus Strep-tagged Csm3 proteins (Csm3_StrepN) were subjected to further characterization. Individual Csm3-N-Strep protein was purified using StrepTrap affinity column. Csm3-N-Strep and Csm complexes eluted from the columns were dialysed against 10 mM Tris-HC1 (pH 8.5) buffer containing 300 mM NaC1, 1 mM DTT, 0.1 mM EDTA, and 50% (v/v) glycerol, and stored at -20°C.

The composition of the isolated Csm-40 and Csm-72 complexes was analysed by SDS-PAGE and the sequence of Csm proteins was further confirmed by the mass spectrometry of tryptic digests. In order to estimate the stoichiometry of Csm complexes, protein bands in SDS-PAGE were quantified by densitometric analysis taking a count the different staining of Cas/Csm proteins. The molecular weights of the Csm complexes were estimated by dynamic light scattering (DLS) using Zetasizer µV (Malvern) and respective software. For DLS analysis Csm-40 and Csm-72 samples were analysed in a Chromatography buffer at 0.36 mg/ml and 0.6 mg/ml concentrations, respectively. Csm complex concentrations were estimated by Pierce 660nm Protein Assay (Thermo Scientific) using bovine serum albumin (BSA) as a reference protein. Conversion to molar concentration was performed assuming that the Csm-72 stoichiometry is Cas10₁:Csm2₆:Csm3₁₀:Csm4₁:crRNA72₁ and the Csm-40 stoichiometry is Cas10₁ :Csm2₃:Csm3₅:Csm4ᵢ :Csm5ᵢ :crRNA401.

### Bioinformatic analysis and mutagenesis of Csm3

Putative active site residues of Csm3 were identified from multiple alignment of Csm3/Cmr4. Csm3 mutants were constructed using quick change mutagenesis and purified as described.

### Mutagenesis of Csm3

The Csm3 mutants H19A, D33A, D100A, EI 19A, E123A and E139A were obtained by the Quick Change Mutagenesis (QCM) Protocol (Zheng et al., 2004). First, a 3.0 kb DNA fragment containing *csm2* and *csm3* genes was subcloned from pCas/Csm plasmid into the pUC18 vector pre-cleaved with Sphl and Kpnl. The resulting plasmid pUC18_Csm2_Csm3 was used for Csm3 QCM mutagenesis. After QCM, the same fragment containing mutated versions of the Csm3 gene was transferred back into the pCas/Csm vector using Ndel and Spel sites, reconstituting the gene cassette. Sequencing of the entire cloned DNA fragment for each mutant confirmed that only the designed mutation had been introduced. Csm-40 complexes containing Csm3 mutants were isolated following the procedures described for the wt StCsm-40 (see above). D100A mutant StCsm-40 was purified only using the affinity chromatography.

### Extraction, HPLC purification and ESI-MS analysis of crRNA

NAs co-purified with Csm-40 and Csm-72 were isolated using phenol:chloroform:isoamylalcohol (25:24:1, v/v/v) extraction and precipitated with isopropanol. Purified NAs were incubated with 0.8 U DNase I or 8 U RNase I (Thermo Scientific) for 30 min at 37°C. NAs were separated on a denaturing 15% polyacrylamide gel (PAAG) and visualized by SybrGold (Invitrogen) staining.

Ion-pair reversed-phased-HPLC purified crRNA architecture was determined using denaturing RNA chromatography in conjunction with electrospray ionization mass spectrometry (ESI-MS) as described in (Sinkunas et al., 2013).

All samples were analyzed by ion-pair reversed-phased-HPLC (Dickman and Homby, 2006; Waghmare et al., 2009) on an Agilent 1100 HPLC with UV260nm detector (Agilent) using a DNAsep column 50 mm x 4.6 mm I. D. (Transgenomic). The chromatographic analysis was performed using the following buffer conditions: A) 0.1 M triethylammonium acetate (TEAA) (pH 7.0) (Fluka); B) buffer A with 25% LC MS grade acetonitrile (v/v) (Fisher). The crRNA was obtained by injecting purified intact Csm-40 or Csm-72 at 75°C using a linear gradient starting at 15% buffer B and extending to 60% B in 12.5 mM, followed by a linear extension to 100% B over 2 mM at a flow rate of 1.0 ml/min. Analysis of the 3' terminus was performed by incubating the HPLC-purified crRNA in a final concentration of 0.1 M HC1 at 4°C for 1 hour. The samples were concentrated to 10-20 µ1 on a vacuum concentrator (Eppendorf) prior to ESI-MS analysis.

### ESI-MS analysis of crRNA

Electrospray Ionization Mass spectrometry (ESI-MS) was performed in negative mode using an Amazon Ion Trap mass spectrometer (Bruker Daltonics), coupled to an online capillary liquid chromatography system (Ultimate 3000, Dionex, UK). RNA separations were performed using a monolithic (PS-DVB) capillary column (50 mm x 0.2 mm I.D., Dionex, UK). The chromatography was performed using the following buffer conditions: C) 0.4 M 1,1,1,3,3,3,-Hexafluoro-2-propanol (HFIP, Sigma- Aldrich) adjusted with triethylamine (TEA) to pH 7.0 and 0.1 mM TEAA, and D) buffer C with 50% methanol (v/v) (Fisher). RNA analysis was performed at 50°C with 20% buffer D, extending to 40% D in 5 min followed by a linear extension to 60% D over 8 min at a flow rate of 2µl/min, 250 ng crRNA was digested with 1U RNase A/T1 (Applied Biosystems). The reaction was incubated at 37°C for 4 h. The oligoribonucleotide mixture was separated on a PepMap C-18 RP capillary column (150 mm x 0.3 gm I.D., Dionex, UK) at 50°C using gradient conditions starting at 20% buffer C and extending to 35% D in 3 mins, followed by a linear extension to 60% D over 40 mins at a flow rate of 2 µl/min. The mass spectrometer was operated in negative mode, a capillary voltage was set at -2500 V to maintain capillary current between 30-50 nA , temperature of nitrogen 120°C at a flow rate of 4.0 L/h and N2 nebuliser gas pressure at 0.4 bar. A mass range of 500-2500 m/z was set. Oligoribonucleotides with -2 to -4 charge states were selected for tandem mass spectrometry using collision induced dissociation.

### Small angle X-ray scattering (SAXS) experiments

SAXS data for Csm-40 and Csm-72 were collected at P12 EMBL beam-line at PETRAIII storage ring of DESY synchrotron in Hamburg (Germany). Csm-40 and Csm-72 complexes were measured in 3 different concentrations in buffer containing 20 mM Tris-HCl (pH 8.5 at 25°C), 0.5 M NaCl, 1mM EDTA and 7 mM 2-mercaptoethanol. Data collection, processing and *ab initio* shape modeling details are presented in Table 4 and FIG. 10.

*Ab initio* shape modeling of both complexes was performed with the samples having highest concentration (1.3 mg/ml for Csm-40 and 2.0 mg/ml for Csm-72). Unprocessed scattering data with subtracted buffer scattering, Guinier plots of the low s region of the scattering curves used for the shape determination and P(r) functions of the highest concentration samples of Csm-40 and Csm-72 are presented in FIG. 10. Two-dimensional scattering curves were transformed and distance distribution functions P(r) were calculated using GNOM (Svergun, 1992). At this stage data were truncated to s values 0.15-0.1 A⁻¹ and calculated distance distribution function was used for following *ab initio* modeling. 10 independent bead models for both complexes were generated using DAMMIN (Svergun, 1999). These models were aligned, filtered and averaged based on occupancy using DAMAVER (Volkov and Svergun, 2003). The averaged NSD of superposition of DAMMIN models of Csm-40 complex was 0.563±0.028 (for Csm-72 models averaged NSD is 0.575±0.019), no model was rejected in both cases.

The inertia tensor was calculated for averaged models of both complexes and models were aligned along the largest principal axis so as the end points of both models coincided. After that the protruding part of the longer Csm-72 complex was truncated. Csm-40 model was aligned with truncated Csm-72 models by automatic procedure SUPCOMB (Kozin and Svergun, 2001) producing an NSD value. Then Csm-40 model was shifted along the principal axis of Csm-72 model by the fixed step (5 or 10 A) and again Csm-40 model was aligned by SUPCOMB with the Csm-72 model after truncation of protruding parts. Thus the Csm-40 model was sequentially shifted along the principal axis of Csm-72 model and the best superposition showed the lower NSD value (S. Grazulis, personal communication). MOLSCRIPT (Kraulis, 1991) and RASTER3D (Merritt and Bacon, 1997) programs were used for SAXS models presented in FIGS. 1 and 10.

### DNA and RNA substrates

Synthetic oligodeoxynucleotides were purchased from Metabion. All RNA substrates were obtained by *in vitro* transcription using TranscriptAid T7 High Yield Transcription Kit (Thermo Scientific). A full description of all the DNA and RNA substrates is provided in the Table 5. DNA and RNA substrates were either 5'-labeled with [γ³²P] ATP and PNK or 3'-labeled with [α³²P] cordycepin-5'-triphospate (PerkinElmer) and poly(A) polymerase (Life Technologies) followed by denaturing gel purification.
To assemble DNA oligoduplexes, complementary oligodeoxynucleotides were mixed at 1:1 molar ratio in the Reaction buffer (33 mM Tris-acetate (pH 7.9 at 25°C), 66 mM potassium acetate), heated to 90°C and slowly let to cool to room temperature.

For generation of S3/1-10, S3/14 RNA substrates, first pUC18 plasmids pUC18_S3/1 and pUC18_S3/2, bearing S3/1 or S3/2 sequences were constructed. For this purpose, annealed synthetic DNA oligoduplexes S3/1 or S3/2 were ligated into pUC18 plasmid pre-cleaved with Smal. Engineered plasmids pUC18_S3/1 and pUC18_S3/2 were sequenced to persuade that only copy of DNA duplex was ligated into the vector. Further these plasmids were used as a template to produce different DNA fragments by PCR using appropriate primers containing a T7 promoter in front of the desired RNA sequence. Purified PCR products were used in the in vitro transcription reaction to obtain RNA substrates. S3/11-13 RNAs were prepared by hybridizing two complementary DNA oligonucleotides, containing a T7 promoter in front of the desired RNA sequence followed by *in vitro* transcription.

DNA/RNA hybrids were assembled in similar manner annealing complementary oligodeoxynucleotide to RNA obtained by *in vitro* transcription.

pBR322 plasmid bearing the Tc gene, encoding tetracycline (Tc) resistance protein, was used to produce Tc RNA and ncTc RNA substrates using the same in vitro transcription reaction as described above for S3/1-10, S3/14. Prior to ³²P 5'-labeling RNA substrates were dephosphorylated using FastAP thermosensitive alkaline phosphatase (Thermo Scientific).

### Electrophoretic mobility shift assay

Binding assays were performed by incubating different amounts of Csm complexes with 0.5 nM of ³²P-5'-labeled NA in the Binding buffer (40 mM Tris, 20 mM acetic acid (pH 8.4 at 25°C), 1 mM EDTA, 0.1 mg/ml BSA, 10% (v/v) glycerol). All reactions were incubated for 15 min at room temperature prior to electrophoresis on native 8% (w/v) PAAG. Electrophoresis was carried out at room temperature for 3 h at 6 V/cm using 40 mM Tris, 20 mM acetic acid (pH 8.4 at 25°C), 0.1 mM EDTA as the running buffer. Gels were dried and visualized using a FLA-5100 phosphorimager (Fujifilm). The K_{d} for NA binding by Csm-72 and Csm-40 was evaluated assuming the complex concentration at which half of the substrate is bound as a rough estimate of K_{d} value. For binding competition assay 0.5 nM ³²P-labelled S3/1 RNA was mixed with 0.5-5000 nM of unlabelled competitor NA and 0.3 nM StCsm-40, and analyzed by EMSA.

### Cleavage assay

The Csm-40 reactions were performed at 25°C and contained 20 nM of 5'- or 3'-radiolabeled NA (Table 5) and 62.5 nM (unless stated otherwise) complex in the Reaction buffer (33 mM Tris-acetate (pH 7.9 at 25°C), 66 mM K-acetate, 0.1 mg/ml BSA and 10 mM Mg-acetate). Csm-72 reactions were performed in the same Reaction buffer at 37°C and contained 20 nM of radiolabeled NA and 125 nM of complex unless stated otherwise. Cleavage reactions using minimal StCsm were performed in the same Reaction buffer at 37°C and contained 4 nM of radiolabeled RNA and -15 ng/µ1 of the RNP complex. Reactions were initiated by addition of the Csm complex. The samples were collected at timed intervals and quenched by mixing 10 µl of reaction mixture with 2X RNA loading buffer (Thermo Scientific) followed by incubation for 10 min at 85°C. The reaction products were separated on a denaturing 20% PAAG and visualized by autoradiography. ³²P-5'-labeled RNA Decade marker (Ambion) was used as size marker. To map the cleavage products oligoribonucleotide markers were generated by RNase A (Thermo Scientific, final concentration 10 ng/ml) treatment of RNA substrates for 8 min at 22°C or by alkaline hydrolysis in 50 mM NaHCO₃ (pH 9.5) at 95°C for 5 min.

### Fluorescent microscopy

Transformed *E.coli* cells producing GFP and StCsm were diluted 1:40 from an overnight culture in fresh LB medium and cells were further grown at 37°C for 2h in the presence of 1% IPTG to induce Cas/Csm, GFP and crRNA expression. The GFP transcript degradation was monitored by inspecting GFP fluorescence in *E.coli* cells. For this purpose, an aliquot of bacteria (2µl) was immediately mounted on a thin film of 1.2% agarose (Thermofisher Scientific) on microscope slides and then overlaid with a coverslip (Roth). The cells were immediately imaged by contrast and fluorescence microscopy. Acquisition of contrast and fluorescence images was performed using a Nicon Elipse Ti-U microscope coupled to a Nicon DS-Qi1 camera. The digital images were analyzed with NIS Element v.4.00.00 (Nicon) software. No electronic enhancement or manipulation was applied to the images.

### Phage drop plaque assay

Phage drop plaque assay was conducted using LGC Standards recommendations. Phage drop plaque assay was conducted using LGC Standards recomendations. Briefly, E. coli NovaBlue(DE3) [(endA 1 hsdR17(rₖ₁₂₋m_{K12+}) supE44 thi-1 recAl gyrA96 relAl lac (DE3) FlproA⁺B⁺ lad q ZΔM15::Tn10] (Tet^{R})] was trasformed with wt pCas/Csm (Str^{R}) or D33A Csm3 pCas/Csm (Str^{R}) and pCRISPR_MS2 (Cm^{R}), pCRISPR_S3 (Cm^{R}), or pACYC-Duet-1 (Cm^{R}). *E. coli* cells bearing different sets of plasmids were grown in LB medium with appropriate antibiotics at 37°C to an OD 600 of 0.9 and a 0.4 ml aliquot of bacterial culture was mixed with melted 0.5% soft nutrient agar (45°C). This mixture was poured onto 1.5% solid agar to make double layer agar plates. Both layers of agar contained appropriate antibiotics, 0.1 mM IPTG, 0.1% glucose, 2 mM CaC1₂ and 0.01 mg/ml thiamine. When the top agar hardened, phage stock (5 µl) from a dilution series was delivered on each plate with the bacteria. The plates were examined for cell lysis after overnight incubations at 37°C. NovaBlue(DE3) was used as the indicator for determining the phage titer. pCRISPR_MS2 plasmid bearing the synthetic CRISPR array of five repeats interspaced by four 36-nt spacers targeting the mat, lys, cp, and rep MS2 RNA sequences (GenBank accession number NC001417) was constructed similarly to pCRISPR_S3 (see above).

### Computational sequence and structure analysis

Sequence searches were performed with PSI-BLAST (Altschul et al., 1997) against the nr80 sequence database (the NCBI 'nr' database filtered to 80% identity) using E-value=1 e-03 or a more stringent inclusion threshold. Clustering of homologous sequences according to their mutual similarity was done using CLANS (Frickey and Lupas, 2004). Multiple sequence alignments were constructed with MAFFT (Katoh et al., 2002) using the accuracy-oriented mode (L-INS-i). Homology model for StCsm3 was constructed with HHpred (Söding et al., 2005) using the related structure of *M kandleri* Csm3 (PDB code 4NOL) as a template. The analysis of surface residue conservation was performed using the ConSurf server (Ashkenazy et al., 2010). Electrostatic map of the structure surface was calculated with the APBS (Baker et al., 2001) plugin in PyMol (Schrodinger, 2010). Pictures were prepared with PyMol (Schrodinger, 2010).

### Engineering of single-gene deletion mutants

pCas/Csm plasmid was used as a template to generate the following single-gene deletion mutant variants: pCas/CsmΔCas6, pCas/CsmΔCas10, pCas/CsmΔCsm4, and pCas/CsmΔCsm6'ΔCsm6. To obtain the pCas/CsmΔCas6 variant, pCas/Csm plasmid was cleaved with Bsp1407I, the remaining sticky ends were blunted, phosphorylated (using "Fast DNA End Repair Kit" from Thermo Scientific), and ligated. This resulted into the *cas6* gene truncation to 67 codons. To obtain pCas/CsmΔCas10, a Bsp119I fragment was excised from the pCas/Csm plasmid. The re-ligated plasmid resulted in the *cas10* gene truncation to 185 codons. To obtain pCas/CsmΔCsm4, pCas/Csm was cleaved with Spel and Eco31I, blunt-ended and re-ligated. This resulted in Csm4 ORF trunction to 41 codons. To obtain pCas/CsmΔCsm6'ΔCsm6, pCas/Csm was cleaved with Ppil and XmaJI, and resulting larger DNA fragment blunt-ended and subjected to ligation. This resulted in the Csm6' ORF truncation to 324 codons and elimination of Csm6 ORF. To obtain pCas/CsmΔCsm5, pUC18_Csm5_Csm6'_Csm6 plasmid was constructed by subcloning a 2.7 kb DNA fragment containing *csm5, csm6',* and *csm6* genes from pCas/Csm plasmid into pUC18 vector, pre-cleaved with Sphl and Kpnl. pUC18_Csm5_Csm6'_Csm6 was cleaved with Swal and BsaAI, the resulting larger DNA fragment was ligated to yield pUC18_ ΔCsm5_Csm6'_Csm6 plasmid, containing a frameshift mutation at the start of *csm5* gene. The Sphl and Pacl fragment containing *Δcsm5, csm6',* and *csm6* was subcloned into the pCas/Csm plasmid to yield pCas/CsmΔCsm5.

pCas/CsmΔCsm2 and pCas/CsmΔCsm3 were engineered using pUC18_Csm2_Csm3 plasmid (see section Mutagenesis of Csm3). To obtain the pCas/CsmΔCsm2, pUC18_Csm2_Csm3 was cleaved with BspMI and Aflll, while to obtain pCas/CsmΔCsm3, pUC18_Csm2_Csm3 was cleaved with Clal and Xhol. The resulting large DNA fragments were then blunted, phosphorylated, and ligated and subcloned into pCas/Csm via Ndel and Spel sites. This resulted in the Csm2 ORF truncation to 70 codons, and Csm3 ORF truncation to 57 codons. Full sequencing of cloned DNA fragments confirmed their identity to the expected sequences. In all cases the deletions were executed in such a way that ribosome binding sites for other genes would not be disrupted. StCsm-40 and StCsm-72 complexes lacking single deleted protein were isolated following the procedures described for the wt StCsm-40 (see above).

### References:

Altschul, S.F., Madden, T.L., Schaffer, A.A., Zhang, J., Zhang, Z., Miller, W., and Lipman, D.J. (1997). Gapped BLAST and PSI-BLAST: a new generation of protein database search programs. Nucleic Acids Res 25, 3389-3402.
Ashkenazy, H., Erez, E., Martz, E., Pupko, T., and Ben-Tal, N. (2010). ConSurf 2010: calculating evolutionary conservation in sequence and structure of proteins and nucleic acids. Nucleic Acids Res 38, W529-533.
Baker, N.A., Sept, D., Joseph, S., Holst, M.J., and McCammon, J.A. (2001). Electrostatics of nanosystems: application to microtubules and the ribosome. Proc Natl Acad Sci U S A 98, 10037-10041.
Dickman, M.J., and Hornby, D.P. (2006). Enrichment and analysis of RNA centered on ion pair reverse phase methodology. RNA 12, 691-696.
Fischer, H., de Oliveira Neto, M., Napolitano, H., Polikarpov, I., and Craievich, A. (2010). Determination of the molecular weight of proteins in solution from a single small-angle X-ray scattering measurement on a relative scale. J Appl Crystallogr 43, 101-109.
Frickey, T., and Lupas, A. (2004). CLANS: a Java application for visualizing protein families based on pairwise similarity. Bioinformatics 20, 3702-3704.
Hale, C.R., Zhao, P., Olson, S., Duff, M.O., Graveley, B.R., Wells, L., Terns, R.M., and Terns, M.P. (2009). RNA-guided RNA cleavage by a CRISPR RNA-Cas protein complex. Cell 139, 945-956. Hatoum-Aslan, A., Samai, P., Maniv, I., Jiang, W., and Marraffini, L.A. (2013). A ruler protein in a complex for antiviral defense determines the length of small interfering CRISPR RNAs. The Journal of biological chemistry 288, 27888-27897.
Horvath, P., and Barrangou, R. (2010). CRISPR/Cas, the immune system of bacteria and archaea. Science 327, 167-170.
Hrle, A., Su, A.A., Ebert, J., Benda, C., Randau, L., and Conti, E. (2013). Structure and RNA-binding properties of the type III-A CRISPR-associated protein Csm3. RNA biology 10, 1670-1678.
Katoh, K., Misawa, K., Kuma, K., and Miyata, T. (2002). MAFFT: a novel method for rapid multiple sequence alignment based on fast Fourier transform. Nucleic Acids Res 30, 3059-3066.
Kozin, M.B., and Svergun, D.I. (2001). Automated matching of high- and low-resolution structural models. J Appl Crystallogr 33, 33-41.
Kraulis, P.J. (1991). MOLSCRIPT: A program to produce both detailed and schematic plots of protein structures. J Appl Crystallogr 24, 945-950.
Makarova, K., Slesarev, A., Wolf, Y., Sorokin, A., Mirkin, B., Koonin, E., Pavlov, A., Pavlova, N., Karamychev, V., Polouchine, N., et al. (2006). Comparative genomics of the lactic acid bacteria. Proc Natl Acad Sci U S A 103,15611-15616.
Marraffini, L.A., and Sontheimer, E.J. (2008). CRISPR interference limits horizontal gene transfer in staphylococci by targeting DNA. Science 322, 1843-1845.
Merritt, E.A., and Bacon, D.J. (1997). Raster3D: Photorealistic molecular graphics. Methn Enzymol 277, 505-524.
Millen, A.M., Horvath, P., Boyaval, P., and Romero, D.A. (2012). Mobile CRISPR/Cas-mediated bacteriophage resistance in Lactococcus lactic. PLoS One 7, e51663.
Petoukhov, M.V., Franke, D., Shkumatov, A.V., Tria, G., Kikhney, A.G., Gajda, M., Gorba, C., Mertens, H.D.T., Konarev, P.V., and Svergun, D.I. (2012). New developments in the ATSAS program package for small-angle scattering data analysis. J Appl Crystallogr 45, 342-350.
Rouillon, C., Zhou, M., Zhang, J., Politis, A., Beilsten-Edmands, V., Cannone, G., Graham, S., Robinson, C.V., Spagnolo, L., and White, M.F. (2013). Structure of the CRISPR interference complex CSM reveals key similarities with cascade. Mol Cell 52, 124-134.
Schrodinger, LLC (2010). The PyMOL Molecular Graphics System, Version 1.3r1.
Söding, J., Biegert, A., and Lupas, A.N. (2005). The HHpred interactive server for protein homology detection and structure prediction. Nucleic Acids Res 33, W244-248.
Staals, R.H., Agari, Y., Maki-Yonekura, S., Zhu, Y., Taylor, D.W., van Duijn, E., Barendregt, A., Vlot, M., Koehorst, J.J., Sakamoto, K., et al. (2013). Structure and activity of the RNA-targeting Type III-B CRISPR-Cas complex of Thermus thermophilus. Mol Cell 52, 135-145.
Svergun, D.I. (1992). Determination of the regularization parameter in indirect-transform methods using perceptual criteria. J Appl Crystallogr 25, 495-503
Svergun, D.I. (1999). Restoring low resolution structure of biological macromolecules from solution scattering using simulated annealing. Biophysical journal 2879-2886.
Volkov, V.V., and Svergun, D.I. (2003). Uniqueness of it ab initio shape determination in small-angle scattering. J Appl Crystallogr 36, 860-864.
Waghmare, S.P., Pousinis, P., Homby, D.P., and Dickman, M.J. (2009). Studying the mechanism of RNA separations using RNA chromatography and its application in the analysis of ribosomal RNA and RNA:RNA interactions. Journal of chromatography. A 1216, 1377-1382.
Zhang, J., Rouillon, C., Kerou, M., Reeks, J., Brugger, K., Graham, S., Reimann, J., Cannone, G., Liu, H., Albers, S.V., et al. (2012). Structure and mechanism of the CMR complex for CRISPR-mediated antiviral immunity. Mol Cell 45, 303-313.
Zheng, L., Baumann, U., and Reymond, J.L. (2004). An efficient one-step site-directed and site-saturation mutagenesis protocol. Nucleic Acids Res 32, el 15.
Brouns, S.J., Jore, M.M., Lundgren, M., Westra, E.R., Slijkhuis, R.J., Snijders, A.P., Dickman, M.J., Makarova, K.S., Koonin, E.V., and van der Oost, J. (2008). Small CRISPR RNAs guide antiviral defense in prokaryotes. Science 321, 960-964.
Carte, J., Wang, R., Li, H., Terns, R.M., and Terns, M.P. (2008). Cas6 is an endoribonuclease that generates guide RNAs for invader defense in prokaryotes. Genes & development 22, 3489-3496.
Deng, L., Garrett, R.A., Shah, S.A., Peng, X., and She, Q. (2013). A novel interference mechanism by a type IIIB CRISPR-Cmr module in Sulfolobus. Molecular microbiology 87, 1088-1099.
Gasiunas, G., Barrangou, R., Horvath, P., and Siksnys, V. (2012). Cas9-crRNA ribonucleoprotein complex mediates specific DNA cleavage for adaptive immunity in bacteria. Proc Natl Acad Sci U S A 109, E2579-2586.
Hale, C.R., Zhao, P., Olson, S., Duff, M.O., Graveley, B.R., Wells, L., Terns, R.M., and Terns, M.P. (2009). RNA-guided RNA cleavage by a CRISPR RNA-Cas protein complex. Cell 139, 945-956.
Hatoum-Aslan, A., Maniv, I., and Marraffini, L.A. (2011). Mature clustered, regularly interspaced, short palindromic repeats RNA (crRNA) length is measured by a ruler mechanism anchored at the precursor processing site. Proc Natl Acad Sci U S A 108, 21218-21222.
Hatoum-Aslan, A., Maniv, I., Samai, P., and Marraffini, L.A. (2014). Genetic characterization of antiplasmid immunity through a type III-A CRISPR-Cas system. J Bacteriol 196, 310-317.
Hatoum-Aslan, A., Samai, P., Maniv, I., Jiang, W., and Marraffini, L.A. (2013). A ruler protein in a complex for antiviral defense determines the length of small interfering CRISPR RNAs. The Journal of biological chemistry 288, 27888-27897.
Hrle, A., Su, A.A., Ebert, J., Benda, C., Randau, L., and Conti, E. (2013). Structure and RNA-binding properties of the type III-A CRISPR-associated protein Csm3. RNA biology 10, 1670-1678.
Jinek, M., Chylinski, K., Fonfara, I., Hauer, M., Doudna, J.A., and Charpentier, E. (2012). A programmable dual-RNA-guided DNA endonuclease in adaptive bacterial immunity. Science 337, 816-821.
Makarova, K.S., Aravind, L., Wolf, Y.I., and Koonin, E.V. (2011a). Unification of Cas protein families and a simple scenario for the origin and evolution of CRISPR-Cas systems. Biology direct 6, 38.
Makarova, K.S., Haft, D.H., Barrangou, R., Brouns, S.J., Charpentier, E., Horvath, P., Moineau, S., Mojica, F.J., Wolf, Y.I., Yakunin, A.F., et al. (2011b). Evolution and classification of the CRISPR-Cas systems. Nature reviews. Microbiology 9, 467-477.
Marraffini, L.A., and Sontheimer, E.J. (2008). CRISPR interference limits horizontal gene transfer in staphylococci by targeting DNA. Science 322, 1843-1845.
Marraffini, L.A., and Sontheimer, E.J. (2010). CRISPR interference: RNA-directed adaptive immunity in bacteria and archaea. Nature reviews. Genetics 11, 181-190.
Millen, A.M., Horvath, P., Boyaval, P., and Romero, D.A. (2012). Mobile CRISPR/Cas-mediated bacteriophage resistance in Lactococcus lactis. PLoS One 7, e51663.
Mojica, F.J., Diez-Villasenor, C., Garcia-Martinez, J., and Almendros, C. (2009). Short motif sequences determine the targets of the prokaryotic CRISPR defence system. Microbiology 155, 733-740.
Olsthoorn, R., and van Duin, J. (2011). Bacteriophages with ssRNA. In eLS (John Wiley & Sons Ltd, Chichester).
Rouillon, C., Zhou, M., Zhang, J., Politis, A., Beilsten-Edmands, V., Cannone, G., Graham, S., Robinson, C.V., Spagnolo, L., and White, M.F. (2013). Structure of the CRISPR interference complex CSM reveals key similarities with cascade. Mol Cell 52, 124-134.
Sinkunas, T., Gasiunas, G., Waghmare, S.P., Dickman, M.J., Barrangou, R., Horvath, P., and Siksnys, V. (2013). In vitro reconstitution of Cascade-mediated CRISPR immunity in Streptococcus thermophilus. EMBO J 32, 385-394.
Spilman, M., Cocozaki, A., Hale, C., Shao, Y., Ramia, N., Terns, R., Terns, M., Li, H., and Stagg, S. (2013). Structure of an RNA silencing complex of the CRISPR-Cas immune system. Mol Cell 52, 146-152.
Staals, R.H., Agari, Y., Maki-Yonekura, S., Zhu, Y., Taylor, D.W., van Duijn, E., Barendregt, A., Vlot, M., Koehorst, J.J., Sakamoto, K., et al. (2013). Structure and activity of the RNA-targeting Type III-B CRISPR-Cas complex of Thermus thermophilus. Mol Cell 52, 135-145.
Sternberg, S.H., Redding, S., Jinek, M., Greene, E.C., and Doudna, J.A. (2014). DNA interrogation by the CRISPR RNA-guided endonuclease Cas9. Nature 507, 62-67.
Szczelkun, M.D., Tikhomirova, M.S., Sinkunas, T., Gasiunas, G., Karvelis, T., Pschera, P., Siksnys, V., and Seidel, R. (2014). Direct observation of R-loop formation by single RNA-guided Cas9 and Cascade effector complexes. Proc Natl Acad Sci U S A 111, 9798-9803.
Terns, R.M., and Terns, M.P. (2014). CRISPR-based technologies: prokaryotic defense weapons repurposed. Trends in genetics : TIG 30, 111-118.
Westra, E.R., van Erp, P.B., Kunne, T., Wong, S.P., Staals, R.H., Seegers, C.L., Bollen, S., Jore, M.M., Semenova, E., Severinov, K., et al. (2012). CRISPR immunity relies on the consecutive binding and degradation of negatively supercoiled invader DNA by Cascade and Cas3. Mol Cell 46, 595-605.
Wiedenheft, B., Lander, G.C., Zhou, K., Jore, M.M., Brouns, S.J., van der Oost, J., Doudna, J.A., and Nogales, E. (2011). Structures of the RNA-guided surveillance complex from a bacterial immune system. Nature 477, 486-489.
Zhang, J., Rouillon, C., Kerou, M., Reeks, J., Brugger, K., Graham, S., Reimann, J., Cannone, G., Liu, H., Albers, S.V., et al. (2012). Structure and mechanism of the CMR complex for CRISPR-mediated antiviral immunity. Mol Cell 45, 303-313.

The embodiments shown and described in the specification are only specific embodiments of inventors who are skilled in the art and are not limiting in any way.

**Table 1 (related to FIG. 1). Protein identified following mass spectrometry analysis of StCsm-72.**

| **Protein** | **Mass (Da)** | **Score** | **Coverage (%)** | **Peptides** |
|---|---|---|---|---|
| Cas10 | 86891 | 1076 | 36 | LAYYLTR GDYAAIATR VYINQFASDK TVETLVQFEK YFKPTVLNLK YHMANYQSDK HNYKEDLFTK LYVAFGWGSFAAK DSISLFSSDYTFK DIMSELNSPESYR |
| Csm3 | 24541 | 768 | 46 | ITAEANPR FENTIDR TLNELLTAEV AT11/FGNYDVK LLELDYLGGSGSR LKATTVFGNYDVK |
| Csm4 | 33727 | 584 | 33 | KQDLYK IFSALVLESLK DGNLYQVATTR HDQIDQSVDVK |
| Cas6 | 28240 | 197 | 16 | LVFTFK LIFQSLMQK |
| Csm2 | 14817 | 186 | 21 | AQILEALK VQFVYQAGR |
| Csm5 | 41013 | 138 | 12 | LISFLNDNR NHESFYEMGK |

**Table 2 (related to FIG. 1): Proteins identified following mass spectrometry analysis of StCsm-40.**

| **Protein** | **Mass (Da)** | **Score** | **Coverage (%)** | **Peptides** |
|---|---|---|---|---|
| Cas10 | 86891 | 1149 | 30 | LAYYLTR GDYAAIATR VYINQFASDK YFKPTVLNLK YFFNHQDER YHMANYQSDK HNYKEDLFTK LYVAFGWGSFAAK DSISLFSSDYTFK DIMSELNSPESYR IDLFYGALLHDIGK DFNQFLLANFQTR FITNVYDDKLEQIR EKIDLFYGALLHDIGK GNEKDSISLFSSDYTFK IWDTYTNQADIFNVFGAQTDK SKPNFASATYEPFSKGDYAAIATR IWDTYTNQADIFNVFGAQTDKR HALVGADWFDEIADNQVISDQIR |
| Csm3 | 24541 | 801 | 57 | ITAEANPR FENTIDR TLNELLTAEV ATTVFGNYDVK LLELDYLGGSGSR LKATTVFGNYDVK VAEKPSDDSDILSR DPITNLPIIPGSSLK SYTEVKFENTIDR DAFLSNADELDSLGVR FENTIDRITAEANPR NSTFDFELIYEITDENENQVEEDFK |
| Csm4 | 33727 | 554 | 33 | KQDLYK IFSALVLESLK DGNLYQVATTR HDQIDQSVDVK SSGFAFSHATNENYR FELDIQNIPLELSDR FELDIQNIPLELSDRLTK NQPHKDGNLYQVATTR SSGF[?][?][?][?]LDIQNIPLELSDR |
| Csm6 | 28240 | 171 | 16 | LVFTFK LIFQSLMQK RIDHPAQDLAVK SQGSYVIFPSMR |
| Csm2 | 14817 | 110 | | AQILEALK VQFVYQAGR |
| Csm5 | 41013 | 965 | 50 | WDYSAK QADGILQR EFIYENK FYFPDMGK TILMNTTPK KFYFPDMGK |
| | | | | VSDSKPFDNK LISFLNDNR NHESFYEMGK EYDDLFNAIR WNNENAVNDFGR GKEYDDLFNAIR KGKEYDDLFNAIR IEFEITTTTDEAGR LSLLTLAPIHIGNGEK DAFGNPYIPGSSLK LAEKFEAFLIQTRPNAR |

**Table 3 (related to FIG. 1): Mw estimations for StCsm-40 and StCsm-72 by different methods.**

| | SDS-PAGE, kDa* | DLS, kDa** | Mo W server, kDa*** | Porod volume, kDa*** | DAMMIN models, kDa**** |
|---|---|---|---|---|---|
| Csm-40 | 344.8 | 305±75 | 302±9 | 282±15 | 347.5 |
| Csm-72 | 486.2 | 523±128 | 425±15 | 350±9 | 465.6 |

| | | | | | |
|---|---|---|---|---|---|
| * Molecular mass calculated from evaluation of the complex composition by densitometric analysis of the SDS-PAGE gels. ** Molecular mass calculated from dynamic light scattering (DLS) analysis. *** Molecular mass calcualted from the SAXS data by the method described in (Fischer et al., 2010) using the SAXS Mo W program run on the server http://www.if.sc.usp.br/∼saxs/saxsmow.html. **** Molecular mass was estiamted using the Porod volumes calculated from SAXS data and excluded volumes of DAMMIN models as described in (Petoukhov et al., 2012). | | | | | |

**Table 4 (related to FIG. 1): SAXS data collection details and structural parameters of StCsm-40 and StCsm-72 complexes.**

| **Data collection parameters** | | | | | | |
|---|---|---|---|---|---|---|
| Beam line | P12 | | | | | |
| Wavelength, nm | 0.124 | | | | | |
| Sample to detector distance, m | 3.1 | | | | | |
| Detector | Pilatus 2M | | | | | |
| s range, nm⁻¹ | 0.975786 - 4.665330 | | | | | |
| exposure time of each frame, s | 0.05 | | | | | |
| Frames collected | 20 | | | | | |
| Sample storage temperature, °C | 10 | | | | | |
| Cell temperature, °C | 20 | | | | | |

| **Structural parameters** | | | | | | |
|---|---|---|---|---|---|---|
| | **Csm-40** | | | **Csm-72** | | |
| Sample concentrations, mg/ml | 0.13 | 0.52 | 1.34 | 0.20 | 0.65 | 2.00 |
| Guinier range (first-last point) as calculated bv AUTORG | 14-53 | 26-55 | 19-52 | 8-35 | 21-39 | 11-34 |
| P(r) calculation range, A^{°-1} | 0.0114-0.2006 | 0.0114-0.2006 | 0.0117-0.1739 | 0.0089-0.1076 | 0.0108-0.1076 | 0.0084-0.1049 |
| Real space Rg, calculated by GNOM, ^{°A} | 63.59±0.414 | 62.80±0.329 | 63.20±0.163 | 83.82±0.545 | 81.40±0.333 | 83.14±0.287 |
| Real space Rg calculated by DATGNOM, ^{°A} | 64.02 | 62.35 | 63.26 | 84.15 | 81.69 | 84.51 |
| Reciprocal space Rg calculated by DATGNOM, | 68.08 | 58.04 | 61.34 | 81.51 | 79.71 | 83.79 |
| Dmax as parameter for GNOM, ^{°A} | 210 | 208 | 215 | 275 | 265 | 280 |
| Dmax calcutaled bv DATGNOM, ^{°A} | 233.2 | 203.1 | 214.7 | 279.2 | 267.0 | 293.3 |
| Porod volume estimated by DATPOROD, A^{°3} | 452186 | 501468 | 485803 | 611618 | 589997 | 581121 |
| Excluded volume of DAMMIN models, A^{°3} (10 models averaged) | | | 590770±5209 | | | 791440±11366 |

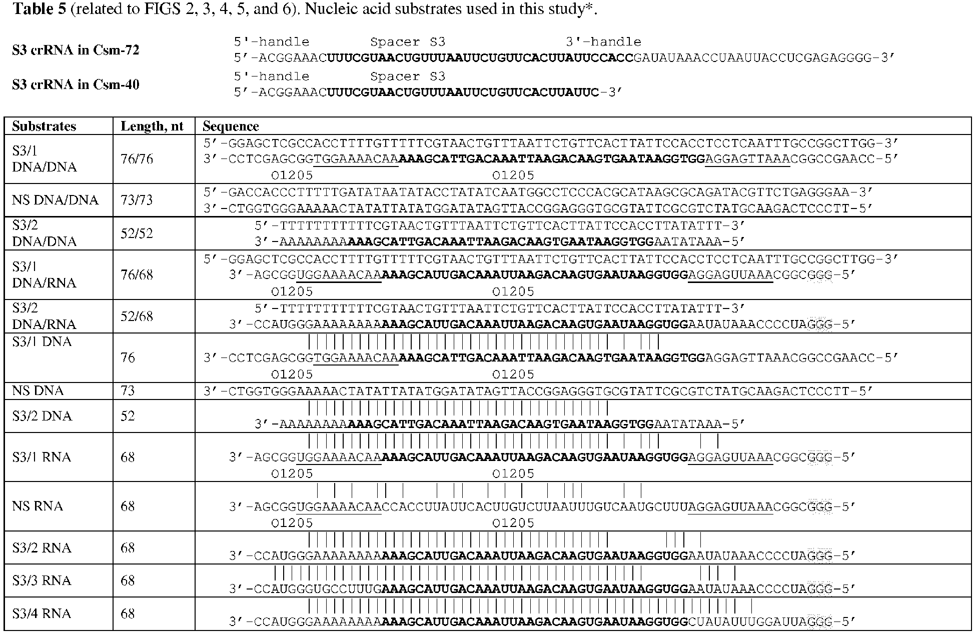

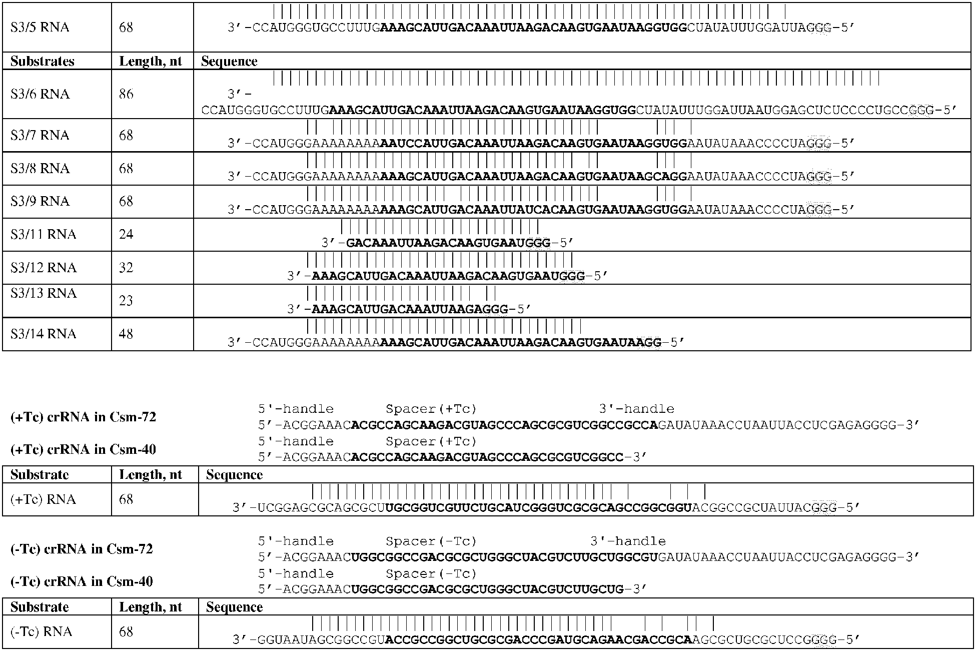

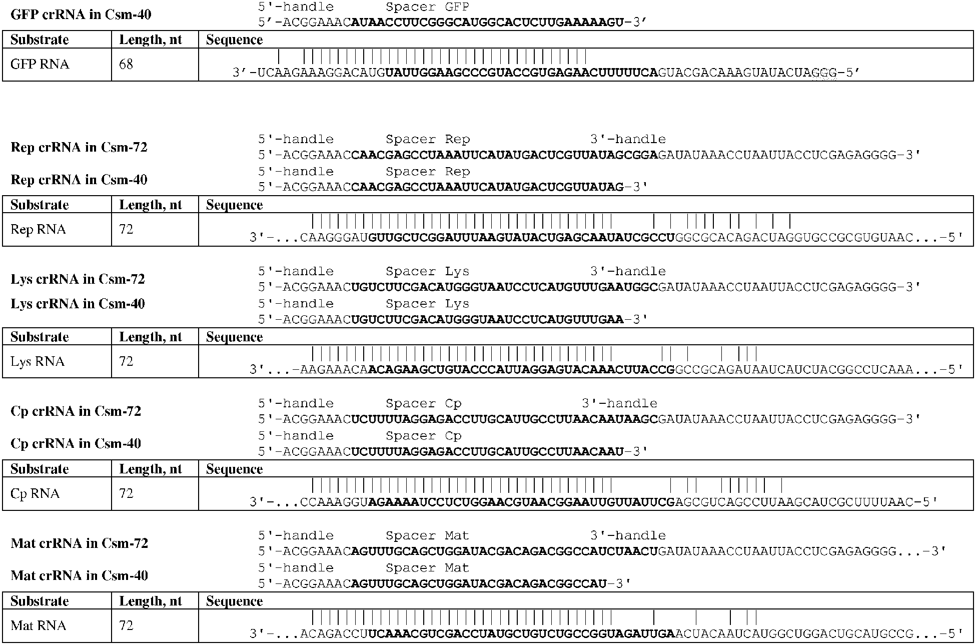

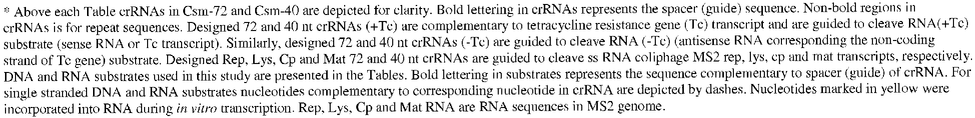

## Claims

1. A composition comprising an engineered Type III-A CRISPR-Cas (StCsm) complex of *Streptococcus thermophilus* consisting of at least crRNA, Csm4, and Csm3, wherein 1 to 10 Csm3 subunits are present, where the crRNA comprises a 5' handle and a spacer, and optionally a 3' handle, wherein the spacer is complementary to a region of a target RNA, and wherein the crRNA is programmed to guide the StCsm complex to a selected site in a target RNA molecule, and wherein the StCsm complex is capable of shredding the target RNA molecule under suitable conditions.

2. A method for programmable shredding of RNA *in vitro* using a Type III-A CRISPR-Cas (StCsm) complex of *Streptococcus thermophilus* according to claim 1, for site specific RNA cleaving under cleavage facilitating conditions.

3. The method according to claim 2 generating a plurality of cleavage fragments in the target RNA and wherein cleavage sites are at 6 nucleotide intervals.

4. The method according to claim 2 wherein 1 to 10 Csm3 subunits which are responsible for an endoribonuclease activity of the complex are present in the StCsm complex.

5. A method for programmable RNA knock-out or knock-down *in vitro* comprising exposing an RNA to be knocked-out or knocked-down to a Type III-A *Streptococcus thermophilus* Csm (III-A) (StCsm) complex according to claim 1, under conditions facilitating RNA knock-out or knock-down.

6. The method according to claim 5 wherein the Csm3 contains a mutation which inactivates the endoribonuclease activity, and the method results in RNA knock-down.

7. The composition according to claim 1 for use in programmable RNA shredding *in vitro* or *in vivo.*

8. The composition according to claim 1 for use in RNA knock-out or knock-down *in vitro* or *in vivo.*

## Patentansprüche

1. Zusammensetzung, umfassend einen gentechnisch bearbeiteten Typ III-A-CRISPR-Cas (StCsm)-Komplex von *Streptococcus thermophilus,* bestehend mindestens aus crRNA, Csm4 und Csm3, wobei 1 bis 10 Csm3-Untereinheiten vorhanden sind, wo die crRNA ein 5'-Handle und einen Spacer und gegebenenfalls ein 3'-Handle umfasst, wobei der Spacer komplementär zu einer Region einer Target-RNA ist und wobei die crRNA programmiert ist, um den StCsm-Komplex zu einer ausgewählten Stelle in einem Target-RNA-Molekül zu führen, und wobei der StCsm-Komplex in der Lage ist, das Target-RNA-Molekül unter geeigneten Bedingungen zu zerkleinern.

2. Verfahren zum programmierbaren Zerkleinern von RNA *in vitro* unter Verwendung eines Typ III-A-CRISPR-Cas (StCsm)-Komplexes von *Streptococcus thermophilus* nach Anspruch 1 für ortsspezifische RNA-Spaltung unter die Spaltung erleichternden Bedingungen.

3. Verfahren nach Anspruch 2, bei dem eine Vielzahl von Spaltfragmenten in der Target-RNA erzeugt werden und wobei sich Spaltungsstellen in 6 Nucleotid-Intervallen befinden.

4. Verfahren nach Anspruch 2, wobei 1 bis 10 Csm3-Untereinheiten, die für eine Endoribonukleaseaktivität des Komplexes verantwortlich sind, in dem StCsm-Komplex vorhanden sind.

5. Verfahren zum programmierbaren RNA-Knockout oder -Knockdown *in vitro,* umfassend Exponieren einer RNA, die einem Knockout oder Knockdown unterzogen werden soll, einem Typ III-A *Streptococcus thermophilus* Csm (III-A) (StCsm)-Komplex nach Anspruch 1, unter Bedingungen, die ein Knockout oder Knockdown erleichtern.

6. Verfahren nach Anspruch 5, wobei die Csm3 eine Mutation enthält, die die Endoribonukleaseaktivität inaktiviert und das Verfahren zum RNA-Knockdown führt.

7. Verfahren nach Anspruch 1 zur Verwendung im programmierbaren RNA-Zerkleinern *in vitro* oder *in vivo.*

8. Verfahren nach Anspruch 1 zur Verwendung im RNA-Knockout oder -Knockdown *in vitro* oder *in vivo.*

## Revendications

1. Composition comprenant un complexe de CRISPR-Cas (StCsm) de type III-A conçu de *Streptococcus thermophilus* constitué par au moins crARN, Csm4 et Csm3, dans laquelle 1 à 10 sous-unités de Csm3 sont présentes, où le crARN comprend une poignée 5' et un écarteur, et facultativement une poignée 3', dans laquelle l'écarteur est complémentaire à une région d'un ARN cible, et dans laquelle le crARN est programmé pour guider le complexe de StCsm vers un site sélectionné dans une molécule d'ARN cible, et dans laquelle le complexe de StCsm est capable de broyer la molécule d'ARN cible dans des conditions appropriées.

2. Procédé de broyage programmable d'ARN *in vitro* en utilisant un complexe de CRISPR-Cas (StCsm) de type III-A de *Streptococcus thermophilus* selon la revendication 1, pour un clivage d'ARN spécifique à un site dans des conditions facilitant le clivage.

3. Procédé selon la revendication 2, générant une pluralité de fragments de clivage dans l'ARN cible et dans lequel des sites de clivage sont à des intervalles de 6 nucléotides.

4. Procédé selon la revendication 2, dans lequel 1 à 10 sous-unités de Csm3 qui sont responsables d'une activité d'endoribonucléase du complexe sont présentes dans le complexe de StCsm.

5. Procédé d'inactivation ou d'inhibition d'ARN programmable *in vitro* comprenant l'exposition d'un ARN pour être inactivé ou inhibé à un complexe de *Streptococcus thermophilus* Csm (StCsm) de type III-Aa (III-A) selon la revendication 1, dans des conditions facilitant une inactivation ou une inhibition de l'ARN.

6. Procédé selon la revendication 5, dans lequel le Csm3 contient une mutation qui inactive l'activité d'endoribonucléase, et le procédé a pour résultat une inhibition de l'ARN.

7. Composition selon la revendication 1 pour une utilisation dans un broyage d'ARN programmable *in vitro* ou *in vivo.*

8. Composition selon la revendication 1 pour une utilisation dans l'inactivation ou l'inhibition d'ARN *in vitro* ou *in vivo.*
